(19) Europäisches Patentamt / European Patent Office / Office européen des brevets

(11) **EP 4 223 214 A1**

(12) **EUROPEAN PATENT APPLICATION**

(43) Date of publication:
**09.08.2023 Bulletin 2023/32**

(21) Application number: **23155317.3**

(22) Date of filing: **07.02.2023**

(51) International Patent Classification (IPC):
**A61B 5/00** (2006.01)    **A61B 5/11** (2006.01)
**A61B 5/024** (2006.01)

(52) Cooperative Patent Classification (CPC):
**A61B 5/4812; A61B 5/024; A61B 5/02405;
A61B 5/1128**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.02.2022 GB 202201557**

(71) Applicant: **Oxehealth Limited
The Oxford Science Park
Oxford
OX4 4GA (GB)**

(72) Inventors:
• **CARTER, Jonathan Frederick
Oxford, OX4 4GA (GB)**
• **JORGE, Joao Goncalo Malverio
Oxford, OX4 4GA (GB)**
• **JONES, Simon Mark Chave
Oxford, OX4 4GA (GB)**
• **TARASSENKO, Lionel
Oxford, OX4 4GA (GB)**

(74) Representative: **J A Kemp LLP
80 Turnmill Street
London EC1M 5QU (GB)**

(54) **MACHINE LEARNING CLASSIFICATION OF SLEEP STATE**

(57) A method of training a supervised machine-learning algorithm to determine a sleep state of a subject. The method uses training data and training labels in respect of a plurality of subjects derived from video images of the subjects. The training data comprises: at least one measure of subject movement; and at least one cardiorespiratory parameter of the subject. The method comprises training the supervised machine learning algorithm using the training data and the training labels.

Fig. 1

**Description**

The invention relates to methods of classifying a sleep state of a subject, in particular using machine-learning analysis of video images.

Polysomnography is used in the diagnosis of various sleep-related disorders. Subjects are monitored while sleeping, and various measurements of brain activity (the electroencephalogram - EEG), eye movement (electro-oculogram - EOG), and other physiological parameters such as the chin Electromyogram (EMG) are recorded. The resulting measurements can be used to determine a depth of sleep of the subject at various points during the period of monitoring, which in turn can be used for various purposes including identifying medical conditions including narcolepsy and sleep apnoea.

Interpretation of the results of a polysomnography study requires review by a trained scorer. The scorer reviews (either on a computer screen or on a print-out) short 'epochs' of the measurements, for example 30-second epochs, and determines information including a classification of each epoch into one of a number of sleep stages. Typically, five stages are used: wake, rapid-eye movement (REM) sleep, and three categories of non-REM sleep indicating light, intermediate, and deep sleep. The latter are usually denoted N1, N2, and N3 in order of increasing depth of sleep. However, other divisions, particularly of non-REM sleep, are possible.

Interpretation by a trained scorer is time-consuming and labour-intensive, as several hours of measurements must be analysed in epochs of less than a minute each. Therefore, polysomnography studies are typically expensive, time-consuming, and difficult to arrange.

Some efforts have been made to automate and simplify the analysis of sleep, but it is challenging to design an automated system that can reliably categorise sleep states. In particular, it is often difficult for automated systems to distinguish light non-REM sleep (N1 stage) from REM sleep without using both the EOG and the chin EMG, and to distinguish the varying levels of non-REM sleep, depending on the measurements that are used.

It would be desirable to develop automated systems for analysing sleep states which do not require the four channels of EEG, the two channels of EOG and the chin EMG, but rather replace them with much easier-to-use and less expensive camera-based technology, in order to reduce the cost of sleep analysis and increase its availability for medical diagnosis.

According to a first aspect of the invention, there is provided a method of training a supervised machine-learning algorithm to determine a sleep state of a subject, wherein: the method uses training data and training labels in respect of a plurality of subjects derived from video images of the subjects, the training data comprises: at least one measure of subject movement; and at least one cardiorespiratory parameter of the subject, and the method comprises training the supervised machine learning algorithm using the training data and the training labels.

The use of video images as the training data for the machine-learning algorithm is advantageous in allowing for non-contact monitoring of sleep state in subjects. This is more comfortable and convenient for subjects than the multiple sensors worn in traditional polysomnography studies, which include 4 channels of electroencephalography (EEG), two channels of electro-oculography (EOG) and one channel of chin electromyography (EMG).

In some embodiments, the training data in respect of each subject is temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep. These are the standard classifications used in sleep monitoring and so are most useful to use in labelling the training data and will be used in most widely-available sleep data sets.

In some embodiments, each epoch is associated with a respective one of the training labels, the training labels selected from a set of training labels indicating that the sleep state of the epoch is wake or sleep; epochs associated with an input label indicating that the sleep state of the epoch is wake are associated with a training label indicating that the sleep state of the epoch is wake; and epochs associated with an input label indicating that the sleep state of the epoch is REM sleep, light NREM sleep, or deep NREM sleep are associated with a training label indicating that the sleep state of the epoch is sleep. Wake/sleep classification can be useful for applications in subject monitoring when the activity status of the subject is of most interest and it is not necessary to know further detail about their sleep state.

In some embodiments, each epoch is associated with a respective one of the training labels, the training labels selected from a set of training labels indicating that the sleep state of the epoch is wake, REM sleep, or NREM sleep; epochs associated with an input label indicating that the sleep state of the epoch is wake are associated with a training label indicating that the sleep state of the epoch is wake; epochs associated with an input label indicating that the sleep state of the epoch is REM sleep are associated with a training label indicating that the sleep state of the epoch is REM sleep; and epochs associated with an input label indicating that the sleep state of the epoch is light NREM sleep, or deep NREM sleep are associated with a training label indicating that the sleep state of the epoch is NREM sleep. Sleep problems often relate to a lack of or disturbances to REM sleep and so distinguishing this state is particularly valuable.

In some embodiments, each epoch is associated with a respective one of the training labels, the training labels selected from a set of training labels indicating that the sleep state of the epoch is wake, REM sleep, light NREM sleep, or deep NREM sleep; epochs associated with an input label indicating that the sleep state of the epoch is wake are associated with a training label indicating that the sleep state of the epoch is wake; epochs associated with an input label indicating that the sleep state of the epoch is REM sleep are associated with a training label indicating that the sleep state of the epoch is REM sleep; epochs associated with an input label indicating that the sleep state of the epoch is light NREM sleep are associated with a training label indicating that the sleep state of the epoch is light NREM sleep; and epochs associated with an input label indicating that the sleep state of the epoch is deep NREM sleep are associated with a training label indicating that the sleep state of the epoch is deep NREM sleep. Distinguishing light and deep NREM sleep is valuable to be able to provide further information on how an individual's sleep pattern is progressing throughout a period of monitoring.

In some embodiments, the set of sleep states further comprises intermediate NREM sleep, and a proportion of the epochs associated with an input label indicating that the sleep state of the epoch is intermediate NREM sleep are associated with the same training label as epochs associated with an input label indicating that the sleep state of the epoch is light NREM sleep. Subjects often do not spend much time in light NREM sleep, leading to a lack of suitable data to train the machine-learning algorithm to recognise light NREM sleep. Intermediate NREM sleep is typically quite similar to light sleep, and so including some intermediate NREM epochs as light sleep can improve the balance of NREM and REM epochs in the light sleep training label, thereby improving reliability of classification.

In some embodiments, the set of sleep states further comprises intermediate NREM sleep; each epoch is associated with a respective one of the training labels, the training labels selected from a set of training labels indicating that the sleep state of the epoch is wake, REM sleep, light NREM sleep, intermediate NREM sleep, or deep NREM sleep; epochs associated with an input label indicating that the sleep state of the epoch is wake are associated with a training label indicating that the sleep state of the epoch is wake; epochs associated with an input label indicating that the sleep state of the epoch is REM sleep are associated with a training label indicating that the sleep state of the epoch is REM sleep; epochs associated with an input label indicating that the sleep state of the epoch is light NREM sleep are associated with a training label indicating that the sleep state of the epoch is light NREM sleep; epochs associated with an input label indicating that the sleep state of the epoch is intermediate NREM sleep are associated with a training label indicating that the sleep state of the epoch is intermediate NREM sleep; and epochs associated with an input label indicating that the sleep state of the epoch is deep NREM sleep are associated with a training label indicating that the sleep state of the epoch is deep NREM sleep. Further distinguishing intermediate NREM provides additional information about the progression of sleep depth in a subject during monitoring.

In some embodiments, each epoch is associated with a respective one of the training labels, the training labels selected from a set of training labels indicating that the sleep state of the epoch is wake, light sleep, or deep sleep; epochs associated with an input label indicating that the sleep state of the epoch is wake are associated with a training label indicating that the sleep state of the epoch is wake; epochs associated with an input label indicating that the sleep state of the epoch is REM sleep or light NREM sleep are associated with a training label indicating that the sleep state of the epoch is light sleep; and epochs associated with an input label indicating that the sleep state of the epoch is deep NREM sleep are associated with a training label indicating that the sleep state of the epoch is deep sleep. Machine learning techniques often have difficulty in separating REM sleep from other sleep states, in particular light NREM sleep. By combining the labels for light NREM sleep and REM sleep, which have many similarities in their characteristics (in particular, in their brain activities), and training the machine learning algorithm on the basis of separating only the depth of sleep, the reliability of separating the sleep depths can be improved.

In some embodiments, the set of sleep states further comprises intermediate NREM sleep, and a proportion of the epochs associated with an input label indicating that the sleep state of the epoch is intermediate NREM sleep are associated with a training label indicating that the sleep state of the epoch is light sleep. Subjects often do not spend much time in light NREM sleep, leading to a lack of suitable data to train the machine-learning algorithm to recognise light NREM sleep. Intermediate NREM sleep is typically quite similar to light sleep, and so including some intermediate NREM epochs as light sleep can improve the balance of NREM and REM epochs in the light sleep training label, thereby improving reliability of classification.

In some embodiments, the machine learning algorithm is a regression algorithm, and the supervised machine-learning algorithm determines the sleep state using a continuous measure of sleep depth, optionally wherein the regression algorithm is a neural network. An advantage of machine-learning against traditional polysomnography techniques is that the sleep state can be quantified more precisely based on its similarity to the different traditional sleep labels.

In some embodiments, the machine learning algorithm is a classification algorithm that classifies the sleep state, optionally wherein the classification algorithm is one of a logistic regression, k-nearest neighbours, a random forest classifier, and a neural network. Classification of this type is more similar to the outcome of traditional polysomnography, and so can allow for more straightforward comparisons of the determined sleep states with previous data, or for diagnoses based on the determined sleep state using existing and traditional techniques.

In some embodiments, the machine learning algorithm is a classification algorithm, optionally one of a logistic regression, k-nearest neighbours, a random forest classifier, and a neural network; the classification algorithm generates scores for each of the training labels representing a confidence of the sleep state being the sleep state indicated by the training label; and the machine-learning algorithm determines the sleep state using a continuous measure of sleep depth, the continuous measure being determined from a weighted combination of the scores for each of the training labels. This allows a continuous measure of sleep depth to be derived from the classification algorithms, which can give a more precise measure of the depth of sleep.

In some embodiments, the at least one measure of subject movement comprises a measure of subject movement in a head region of the subject and/or a measure of movement in a torso region of the subject. In some embodiments, the at least one cardiorespiratory parameter of the subject comprises a respiratory rate of the subject, a variance of the respiratory rate of the subject, a heart rate of the subject, and/or a variance of the heart rate of the subject. These parameters can be gathered from analysis of video images and are indicative of the differences between different sleep states.

In some embodiments, the measure of subject movement and/or the cardiorespiratory parameter of the subject is derived as a rolling average. Rolling averages are advantageous in controlling for transient changes in measured parameters that are not indicative of an underlying change in sleep state.

In some embodiments, the method further comprises deriving the at least one measure of subject movement and the at least one cardiorespiratory parameter of the subject from the video images of the subjects. This ensures that the training data are derived in a manner that is best compatible with the other parts of the method and the training of the machine learning algorithm.

In some embodiments, the step of deriving the at least one cardiorespiratory parameter of the subject comprises deriving heart rate and respiratory rate from the video images of the subjects and deriving the at least one cardiorespiratory parameter from the heart rate and respiratory rate. The heart rate and respiratory rate are quantities most likely to contain cardiorespiratory information relevant to sleep state, and so extracting them first to use for deriving the cardiorespiratory parameters can ensure the cardiorespiratory parameters capture the most relevant information for training the machine learning algorithm to accuracy determine the sleep state.

In accordance with a second aspect of the invention, there is provided a method of determining a sleep state of a test subject comprising applying a machine-learning algorithm to input data from the test subject, wherein: the input data are derived from video images of the test subject; and the input data comprises: at least one measure of subject movement; and at least one cardiorespiratory parameter of the subject; and the machine-learning algorithm outputs a determination of the sleep state of the test subject on the basis of training data and training labels, wherein the training data is in respect of a plurality of training subjects and is derived from video images of the training subjects, the training data comprising for each of the training subjects: at least one measure of subject movement; and at least one cardiorespiratory parameter.

Classifying the sleep state using features derived from video images allows for less intrusive monitoring of the test subject, which is easier to set up and provides more realistic sleep condition from the subject. The use of machine learning for automatic classification also removes the need for less time consuming and expensive human classification of data.

In some embodiments, the training data in respect of each training subject is temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep. These are the standard classifications used in sleep monitoring and so are most useful to use in labelling the training data and will be used in most widely-available sleep data sets.

In some embodiments, the set of sleep states further comprises intermediate NREM sleep. Intermediate NREM is harder to consistently classify than the other states, so may not always be included, but can nonetheless provide useful additional information when present.

In some embodiments, each epoch is associated with a respective one of the training labels, the training labels selected from a set of training labels indicating that the sleep state of the epoch is wake or sleep; epochs associated with an input label indicating that the sleep state of the epoch is wake are associated with a training label indicating that the sleep state of the epoch is wake; and epochs associated with an input label indicating that the sleep state of the epoch is REM sleep, light NREM sleep, or deep NREM sleep are associated with a training label indicating that the sleep state of the epoch is sleep. Wake/sleep classification can be useful for applications in subject monitoring when the activity status of the subject is of most interest and it is not necessary to know further detail about their sleep state.

In some embodiments, each epoch is associated with a respective one of the training labels, the training labels selected from a set of training labels indicating that the sleep state of the epoch is wake, REM sleep, or NREM sleep, wherein epochs associated with an input label indicating that the sleep state of the epoch is wake are associated with a training label indicating that the sleep state of the epoch is wake, epochs associated with an input label indicating that the sleep state of the epoch is REM sleep are associated with a training label indicating that the sleep state of the epoch is REM sleep, epochs associated with an input label indicating that the sleep state of the epoch is light NREM sleep or deep NREM sleep are associated with a training label indicating that the sleep state of the epoch is NREM sleep. Sleep problems often relate to a lack of or disturbances to REM sleep and so distinguishing this state is particularly valuable.

In some embodiments, each epoch is associated with a respective one of the training labels, the training labels selected from a set of training labels indicating that the sleep state of the epoch is wake, REM sleep, light NREM sleep, or deep NREM sleep, wherein epochs associated with an input label indicating that the sleep state of the epoch is wake are associated with a training label indicating that the sleep state of the epoch is wake, epochs associated with an input label indicating that the sleep state of the epoch is REM sleep are associated with a training label indicating that the sleep state of the epoch is REM sleep, epochs associated with an input label indicating that the sleep state of the epoch is light NREM sleep are associated with a training label indicating that the sleep state of the epoch is light NREM sleep, and epochs associated with an input label indicating that the sleep state of the epoch is deep NREM sleep are associated with a training label indicating that the sleep state of the epoch is deep NREM sleep. Distinguishing light and deep NREM sleep is valuable to be able to provide further information on how an individual's sleep pattern is progressing throughout a period of monitoring.

In some embodiments, the set of sleep states further comprises intermediate NREM sleep, and a proportion of the epochs associated with an input label indicating that the sleep state of the epoch is intermediate NREM sleep are associated with the same training label as epochs associated with an input label indicating that the sleep state of the epoch is light NREM sleep. Subjects often do not spend much time in light NREM sleep, leading to a lack of suitable data to train the machine-learning algorithm to recognise light NREM sleep. Intermediate NREM sleep is typically quite similar to light sleep, and so including some intermediate NREM epochs as light sleep can improve the balance of NREM and REM epochs in the light sleep training label, thereby improving reliability of classification.

In some embodiments, the set of sleep states further comprises intermediate NREM sleep; each epoch is associated with a respective one of the training labels, the training labels selected from a set of training labels indicating that the sleep state of the epoch is wake, REM sleep, light NREM sleep, intermediate NREM sleep, or deep NREM sleep, wherein epochs associated with an input label indicating that the sleep state of the epoch is wake are associated with a training label indicating that the sleep state of the epoch is wake, epochs associated with an input label indicating that the sleep state of the epoch is REM sleep are associated with a training label indicating that the sleep state of the epoch is REM sleep, epochs associated with an input label indicating that the sleep state of the epoch is light NREM sleep are associated with a training label indicating that the sleep state of the epoch is light NREM sleep, epochs associated with an input label indicating that the sleep state of the epoch is intermediate NREM sleep are associated with a training label indicating that the sleep state of the epoch is intermediate NREM sleep, and epochs associated with an input label indicating that the sleep state of the epoch is deep NREM sleep are associated with a training label indicating that the sleep state of the epoch is deep NREM sleep. Further distinguishing intermediate NREM provides additional information about the progression of sleep depth in a subject during monitoring.

In some embodiments, each epoch is associated with a respective one of the training labels, the training labels selected from a set of training labels indicating that the sleep state of the epoch is wake, light sleep, or deep sleep, wherein epochs associated with an input label indicating that the sleep state of the epoch is wake are associated with a training label indicating that the sleep state of the epoch is wake, epochs associated with an input label indicating that the sleep state of the epoch is REM sleep or light NREM sleep are associated with a training label indicating that the sleep state of the epoch is light sleep, and epochs associated with an input label indicating that the sleep state of the epoch is deep NREM sleep are associated with a training label indicating that the sleep state of the epoch is deep sleep. Machine learning techniques often have difficulty in separating REM sleep from other sleep states, in particular light NREM sleep. By combining the labels for light NREM sleep and REM sleep, which have many similarities in their characteristics (in particular, in their brain activities), and training the machine learning algorithm on the basis of separating only the depth of sleep, the reliability of separating the sleep depths can be improved.

In some embodiments, the set of sleep states further comprises intermediate NREM sleep, and a proportion of the epochs associated with an input label indicating that the sleep state of the epoch is intermediate NREM sleep are associated with a training label indicating that the sleep state of the epoch is light sleep. Subjects often do not spend much time in light NREM sleep, leading to a lack of suitable data to train the machine-learning algorithm to recognise light NREM sleep. Intermediate NREM sleep is typically quite similar to light sleep, and so including some intermediate NREM epochs as light sleep can improve the balance of NREM and REM epochs in the light sleep training label, thereby improving reliability of classification.

In some embodiments, the machine-learning algorithm is trained using a method according to the first aspect. Some types of machine-learning algorithm can be pre-trained, which can improve performance when the algorithm is run on new subjects. Training the algorithm according to the first aspect provides the advantages discussed in association therewith.

In some embodiments, the at least one measure of subject movement comprises a measure of subject movement in a head region of the subject, and/or a measure of movement in a torso region of the subject. In some embodiments, the at least one cardiorespiratory parameter of the subject comprises a respiratory rate of the subject, a variance of the respiratory rate of the subject, a heart rate of the subject, and/or a variance of the heart rate of the subject. These parameters can be gathered from analysis of video images and are indicative of the differences between different sleep states.

In some embodiments, the determination of the sleep state of the test subject is a value of a continuous measure of sleep depth. An advantage of machine-learning against traditional polysomnography techniques is that the sleep state can be quantified more precisely based on its similarity to the different traditional sleep labels.

In some embodiments, the determination of the sleep state of the test subject is a classification of the sleep state. Classification of this type is more similar to the outcome of traditional polysomnography, and so can allow for more straightforward comparisons of the determined sleep states with previous data, or for diagnoses based on the determined sleep state using existing and traditional techniques.

In some embodiments, the measure of subject movement and/or the cardiorespiratory parameter of the subject is derived as a rolling average. Rolling averages are advantageous in controlling for transient changes in measured parameters that are not indicative of an underlying change in sleep state.

In some embodiments, the method further comprises deriving the at least one measure of subject movement and the at least one cardiorespiratory parameter from the video images of the test subject. This ensures that the training data and/or the input data are derived in a manner that is best compatible with the other parts of the method.

In some embodiments, the step of deriving the at least one cardiorespiratory parameter of the subject comprises extracting heart rate and respiratory rate from the video images of the test subject, and deriving the at least one cardiorespiratory parameter from the heart rate and respiratory rate. The heart rate and respiratory rate are quantities most likely to contain cardiorespiratory information relevant to sleep state, and so extracting them first to use for deriving the cardiorespiratory parameters can ensure the cardiorespiratory parameters capture the most relevant information for training the machine learning algorithm to accuracy determine the sleep state.

According to a third aspect of the invention, there is provided a method of training a supervised machine-learning algorithm to determine a sleep state of a subject, wherein the method uses training data in respect of a plurality of subjects derived from video images of the subjects, the training data in respect of each subject being temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep, and the method comprises training the supervised machine learning algorithm using the training data and a training label associated with each epoch selected from a set of training labels comprising training labels indicating that the sleep state of the epoch is wake, light sleep, or deep sleep, wherein epochs associated with an input label indicating that the sleep state of the epoch is wake are associated with a training label indicating that the sleep state of the epoch is wake, and epochs associated with an input label indicating that the sleep state of the epoch is REM sleep or light NREM sleep are associated with a training label indicating that the sleep state of the epoch is light sleep, epochs associated with an input label indicating that the sleep state of the epoch is deep NREM sleep are associated with a training label indicating that the sleep state of the epoch is deep sleep.

Machine learning techniques often have difficulty in separating all of the conventional divisions of sleep states, in particular separating REM sleep from other sleep states. By combining the labels for light NREM sleep and REM sleep, which have many similarities in their characteristics (in particular, in their brain activities), and training the machine learning algorithm on the basis of separating only the depth of sleep, the reliability of separating the sleep depths can be improved. In addition, the use of video images as the training data for the machine-learning algorithm is advantageous in allowing for non-contact monitoring of sleep state in subjects. This is more comfortable and convenient for subjects than the multiple sensors worn in traditional polysomnography studies, which include 4 channels of electroencephalography (EEG), two channels of electro-oculography (EOG) and one channel of chin electromyography (EMG).

In some embodiments, the set of sleep states further comprises intermediate NREM sleep (usually denoted as N2), and a proportion of the epochs associated with an input label indicating that the sleep state of the epoch is intermediate NREM sleep are associated with a training label indicating that the sleep state of the epoch is light sleep. Subjects often do not spend much time in light NREM sleep, leading to a lack of suitable data to train the machine-learning algorithm to recognise light NREM sleep. Intermediate NREM sleep is typically quite similar to light sleep, and so including some intermediate NREM epochs as light sleep can improve the balance of NREM and REM epochs in the light sleep training label, thereby improving reliability of classification.

In some embodiments, the proportion of the epochs associated with an input label indicating that the sleep state of the epoch is intermediate NREM sleep comprises epochs associated with an input label indicating that the sleep state of the epoch is intermediate NREM sleep that immediately precede or follow an epoch associated with an input label indicating that the sleep state of the epoch is light NREM sleep. Feature values for intermediate NREM epochs adj acent to light NREM epochs are likely to be more similar to light NREM sleep than those in intermediate NREM epochs adj acent to deep NREM epochs. Therefore, these provide better candidates for inclusion in the light sleep training label.

In some embodiment, the set of training labels is a set of three training labels comprising the training labels indicating that the sleep state of the epoch is wake, light sleep, or deep sleep. Limiting to only three labels can reduce the complexity of the training of the machine learning algorithm and lead to improved accuracy.

In some embodiments, the set of sleep states further comprises intermediate NREM sleep; the set of training labels further comprises a training label indicating that the sleep state of the epoch is medium sleep; and epochs associated with an input label indicating that the sleep state of the epoch is intermediate NREM sleep are associated with a training label indicating that the sleep state of the epoch is medium sleep. This allows the algorithm to also identify medium sleep, while maintaining the benefit of combining the REM sleep epoch with light NREM sleep, thereby simplifying the classification.

In some embodiments, the machine learning algorithm is a regression algorithm, and the supervised machine-learning algorithm determines the sleep state using a continuous measure of sleep depth. An advantage of machine-learning against traditional polysomnography techniques is that the sleep state can be quantified more precisely based on its similarity to the different traditional sleep labels.

In some embodiments, the regression algorithm is a neural network. Neural networks been shown to be particularly suitable for the present application.

In some embodiments, the machine learning algorithm is a classification algorithm and the supervised machine-learning algorithm determines the sleep state to be one of wake, light sleep, or deep sleep. Classification of this type is more similar to the outcome of traditional polysomnography, and so can allow for more straightforward comparisons of the determined sleep states with previous data, or for diagnoses based on the determined sleep state using existing and traditional techniques.

In some embodiments, the machine learning algorithm is a classification algorithm and the supervised machine-learning algorithm determines the sleep state to be one of wake, light sleep, medium sleep, or deep sleep. Medium sleep may also be referred to as intermediate sleep. This allows the more precise determination of the depth of sleep even in the case where a classification into discrete states is used.

In some embodiments, the classification algorithm generates scores for each of the training labels representing a confidence of the sleep state being the sleep state indicated by the training label, and the machine-learning algorithm determines the sleep state using a continuous measure of sleep depth, the continuous measure being determined from a weighted combination of the scores for each of the training labels. This allows a continuous measure of sleep depth to be derived from the classification algorithms, which can give a more precise measure of the depth of sleep.

In some embodiments, the classification algorithm is one of a logistic regression, k-nearest neighbours, a random forest classifier, and a neural network. These classification algorithms have been shown to be particularly suitable for the present application.

In some embodiments, the training data comprise at least one feature derived from the video images of the subjects. In some embodiments, the at least one feature comprises a measure of subject movement, for example a measure of subject movement in a head region of the subject and/or a measure of movement in a torso region of the subject. In some embodiments, the at least one feature comprises a cardiorespiratory parameter of the subject, for example a respiratory rate of the subject, a variance of the respiratory rate of the subject, a heart rate of the subject, and/or a variance of the heart rate of the subject. These parameters can be gathered from analysis of video images and are indicative of the differences between different sleep states.

According to a fourth aspect of the invention, there is provided a method of training a supervised machine-learning algorithm to determine a sleep state of a subject, wherein the machine-learning algorithm comprises a first-stage classification algorithm and a second-stage classification algorithm, the method comprising: training the first-stage classification algorithm according to the third aspect of the invention; training the second stage classification algorithm using the training data to further classify the sleep state as NREM sleep or REM sleep when the first stage classification algorithm classifies the sleep state as light sleep.

While it can be challenging to determine a sleep state among all possible sleep states, the present inventors have found that separating light NREM (usually denoted as N1) and REM sleep can be more reliably done by a machine-learning algorithm. Therefore, a two-stage classification process of separating wake, light sleep, and deep NREM sleep followed by separating light NREM and REM sleep can more reliably differentiate these four typical sleep states. When combined with the use of intermediate NREM sleep states and the medium sleep training label, this cascade arrangement allows for the full classification of the sleep state into one of the five, commonly-used R&K states of wake, N1, N2, N3, and REM.

In some embodiments, training the second stage classification algorithm comprises training the second stage classification algorithm using the training data and a second-stage training label associated with each epoch selected from a set of second-stage training labels comprising second-stage training labels indicating that the sleep state of the epoch is NREM sleep or REM sleep, wherein epochs associated with an input label indicating that the sleep state of the epoch is REM sleep are associated with a second-stage training label indicating that the sleep state of the epoch is REM sleep; and epochs associated with an input label indicating that the sleep state of the epoch is light NREM sleep are associated with a second-stage training label indicating that the sleep state of the epoch is NREM sleep.

The purpose of the second stage is to separate REM and light NREM sleep, so training the machine-learning algorithm based on the separation of these sleep states is a straightforward way to differentiate the two states.

In some embodiments, epochs associated with an input label indicating that the sleep state of the epoch is a sleep state other than REM sleep are associated with a second-stage training label indicating that the sleep state of the epoch is NREM sleep. Using all NREM sleep states to train against the REM epochs can be preferable in some situations because it provides additional training data to separate the differences with REM sleep.

In some embodiments, the training data comprise at least one feature derived from the video images of the subjects. In some embodiments, the at least one feature comprises a measure of subject movement, for example a measure of subject movement in a head region of the subject and/or a measure of movement in a torso region of the subject. In some embodiment, the at least one feature comprises a cardiorespiratory parameter of the subject, for example a respiratory rate of the subject, a variance of the respiratory rate of the subject, a heart rate of the subject, and/or a variance of the heart rate of the subject. These parameters can be gathered from analysis of video images and are indicative of the differences between different sleep states.

In some embodiments, at least one of the features is derived as a rolling average. Rolling average filters are advantageous in controlling for transient changes in measured parameters that are not indicative of an underlying change in sleep state.

According to a fifth aspect of the invention, there is provided a method of determining a sleep state of a test subject comprising applying a machine-learning algorithm to input data from the test subject, wherein: the input data are derived from video images of the test subject; and the machine-learning algorithm outputs a determination of the sleep state of the test subject on the basis of training data and training labels, wherein the training data is in respect of a plurality of training subjects and is derived from video images of the training subjects, the training data in respect of each training subject being temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep, the training labels are associated with each epoch and selected from a set of training labels comprising training labels indicating that the sleep state of the epoch is wake, light sleep, or deep sleep, wherein epochs associated with an input label indicating that the sleep state of the epoch is wake are associated with a training label indicating that the sleep state of the epoch is wake, and epochs associated with an input label indicating that the sleep state of the epoch is REM sleep or light NREM sleep are associated with a training label indicating that the sleep state of the epoch is light sleep, epochs associated with an input label indicating that the sleep state of the epoch is deep NREM sleep are associated with a training label indicating that the sleep state of the epoch is deep sleep.

As discussed above, determining sleep state in accordance with the separation of sleep states without attempting to separate REM sleep can provide more reliable determination of sleep depth.

In some embodiments, the machine-learning algorithm is trained using a method according to the third aspect of the invention. Some types of machine-learning algorithm can be pre-trained, which can improve performance when the algorithm is run on new subjects. Training the algorithm according to the third aspect provides the advantages discussed in association therewith.

In some embodiments, the input data comprise at least one feature derived from the video images of the test subject. In some embodiment, the at least one feature comprises a measure of subject movement, for example a measure of subject movement in a head region of the subject and/or a measure of movement in a torso region of the subject. In some embodiment, the at least one feature comprises a cardiorespiratory parameter of the subject, for example a respiratory rate of the subject, a variance of the respiratory rate of the subject a heart rate of the subject, and/or a variance of the heart rate of the subject. These parameters can be gathered from analysis of video images and are indicative of the differences between different sleep states.

In some embodiments, the set of training labels is a set of three training labels comprising the training labels indicating that the sleep state of the epoch is wake, light sleep, or deep sleep. Limiting to only three labels can reduce the complexity of the training the machine learning algorithm and lead to improved accuracy.

In some embodiments, the set of sleep states further comprises intermediate NREM sleep; the set of training labels further comprises a training label indicating that the sleep state of the epoch is medium sleep; and epochs associated with an input label indicating that the sleep state of the epoch is intermediate NREM sleep are associated with a training label indicating that the sleep state of the epoch is medium sleep. This allows the algorithm to also identify medium sleep, while maintaining the benefit of combining the REM sleep epoch with light NREM sleep, thereby simplifying the classification.

In some embodiments, the determination of the sleep state of the test subject is a value of a continuous measure of sleep depth. An advantage of machine-learning against traditional polysomnography techniques is that the sleep state can be quantified more precisely based on its similarity to the different traditional sleep labels.

In some embodiments, the determination of the sleep state of the test subject is a classification of the sleep state as one of wake, light sleep, or deep sleep. Classification of this type is more similar to the outcome of traditional polysomnography, and so can allow for more straightforward comparisons of the determined sleep states with previous data, or for diagnoses based on the determined sleep state using existing and traditional techniques.

In some embodiments, the determination of the sleep state of the test subject is a classification of the sleep state as one of wake, light sleep, medium sleep, or deep sleep. This allows the more precise determination of the depth of sleep even in the case where a classification into discrete states is used.

According to a sixth aspect of the invention, there is provided a method of determining a sleep state of a test subject comprising: determining a sleep state of the test subject using a method according to the fifth aspect of the invention; and if the sleep state is determined to be light sleep, further classifying the light sleep as REM or NREM sleep. As discussed above, the present inventors have found that separating light NREM and REM sleep can be more reliably done by a machine-learning algorithm. Therefore, a two-stage classification process of separating wake, light NREM sleep, and deep NREM sleep followed by separating light NREM and REM sleep can more reliably differentiate these four typical sleep states.

In some embodiments, the input data comprise one or both of a measure of subject movement and a cardiorespiratory parameter of the subject, and the further classification is carried out using the input data. These parameters can be gathered from analysis of video images and are indicative of the differences between light NREM and REM sleep states.

In some embodiments, the further classification is carried out using a second stage machine-learning classification algorithm that outputs a classification of the sleep state as NREM sleep or REM sleep on the basis of the training data and input labels. A machine learning algorithm can efficiently leverage the differences in the training data to reliably separate the light NREM and REM sleep states.

In some embodiments, the second stage classification algorithm uses the training data and a second-stage training label associated with each epoch selected from a set of second-stage training labels comprising second-stage training labels indicating that the sleep state of the epoch is NREM sleep or REM sleep, wherein epochs associated with an input label indicating that the sleep state of the epoch is light NREM sleep are associated with a second-stage training label indicating that the sleep state of the epoch is NREM sleep.

The purpose of the second stage is to separate REM and light NREM sleep, so training the machine-learning algorithm based on the separation of these sleep states is a straightforward way to differentiate the two states.

In some embodiments, epochs associated with an input label indicating that the sleep state of the epoch is a sleep state other than REM sleep are associated with a second-stage training label indicating that the sleep state of the epoch is NREM sleep. Using all NREM sleep states to train against the REM epochs can be preferable in some situations because it provides additional training data to separate the differences with REM sleep.

In some embodiments, the method of determining a sleep state of a test subject comprises applying a machine-learning algorithm trained using a method according to the fourth aspect of the invention to input data from the test subject, the input data derived from video images of the test subject. Some types of machine-learning algorithm can be pre-trained, which can improve performance when the algorithm is run on new subjects. Training the algorithm according to the fourth aspect provides the advantages discussed in association therewith.

In some embodiments, the input data comprise at least one feature derived from the video images of the test subject. In some embodiments, the at least one feature comprises a measure of subject movement, for example a measure of subject movement in a head region of the subject and/or a measure of movement in a torso region of the subject. In some embodiments, the at least one feature comprises a cardiorespiratory parameter of the subject, for example a respiratory rate of the subject, a variance of the respiratory rate of the subject, a heart rate of the subject, and/or a variance of the heart rate of the subject. These parameters can be gathered from analysis of video images and are indicative of the differences between the different sleep states.

In some embodiments, at least one of the features is derived as a rolling average. Rolling averages are advantageous in controlling for transient changes in measured parameters that are not indicative of an underlying change in sleep state.

According to a seventh aspect of the invention, there is provided a method of training a supervised machine-learning algorithm to determine a sleep state of a subject, wherein the method uses training data in respect of a plurality of subjects derived from video images of the subjects, the training data in respect of each subject being temporally divided into a plurality of epochs, each epoch being associated with a respective input label indicating that a sleep state of the epoch is one of a set of sleep states comprising rapid-eye movement (REM) sleep and light non-rapid eye movement (NREM) sleep, and the method comprises training the supervised machine learning algorithm using the training data and a training label associated with each epoch selected from a set of training labels comprising training labels indicating that the sleep state of the epoch is NREM sleep or REM sleep, wherein epochs associated with an input label indicating that the sleep state of the epoch is REM sleep are associated with a second-stage training label indicating that the sleep state of the epoch is REM sleep; and epochs associated with an input label indicating that the sleep state of the epoch is light NREM sleep are associated with a training label indicating that the sleep state of the epoch is NREM sleep.

The use of video images as training data for machine-learning algorithms trained to separate REM and non-REM sleep is advantageous in allowing for non-contact monitoring of sleep state in subjects. This is more comfortable and convenient for subjects than the multiple sensors worn in traditional polysomnography studies.

In some embodiments, epochs associated with an input label indicating that the sleep state of the epoch is a sleep state other than REM sleep are associated with a training label indicating that the sleep state of the epoch is NREM sleep. Using all NREM sleep states to train against the REM epochs can be preferable in some situations because it provides additional training data to separate the differences with REM sleep.

In some embodiments, the training data comprise at least one feature derived from the video images of the subjects. In some embodiment, the at least one feature comprises a measure of subject movement, for example a measure of subject movement in a head region of the subject and/or a measure of movement in a torso region of the subject. In some embodiments, the at least one feature comprises a cardiorespiratory parameter of the subject, for example a respiratory rate of the subject, a variance of the respiratory rate of the subject, a heart rate of the subject, and/or a variance of the heart rate of the subject. These parameters can be gathered from analysis of video images and are indicative of the differences between the different sleep states.

In some embodiments, at least one of the features is derived as a rolling average. Rolling averages are advantageous in controlling for transient changes in measured parameters that are not indicative of an underlying change in sleep state.

According to an eighth aspect of the invention, there is provided a method of determining a sleep state of a subject comprising applying a machine-learning algorithm to input data from the subject, wherein the input data are derived from video images of the subject; and the machine-learning algorithm outputs a determination of the sleep state of the subject as light NREM sleep or REM sleep on the basis of training data and training labels, wherein the training data is in respect of a plurality of subjects and is derived from video images of the subjects, the training data in respect of each subject being temporally divided into a plurality of epochs, each epoch being associated with a respective input label indicating that a sleep state of the epoch is one of a set of sleep states comprising rapid-eye movement (REM) sleep and light non-rapid eye movement (NREM) sleep; the training labels are associated with each epoch and selected from a set of training labels comprising training labels indicating that the sleep state of the epoch is NREM sleep or REM sleep, wherein epochs associated with an input label indicating that the sleep state of the epoch is REM sleep are associated with a second-stage training label indicating that the sleep state of the epoch is REM sleep; and epochs associated with an input label indicating that the sleep state of the epoch is light NREM sleep are associated with a training label indicating that the sleep state of the epoch is NREM sleep.

The use of video images to determine a sleep state of a subject and separate REM and non-REM sleep is advantageous in allowing for non-contact monitoring of sleep state in subjects. This is more comfortable and convenient for subjects than the multiple sensors worn in traditional polysomnography studies.

In some embodiments, the machine-learning algorithm is trained using a method according to the seventh aspect of the invention. Some types of machine-learning algorithm can be pre-trained, which can improve performance when the algorithm is run on new subjects. Training the algorithm according to the seventh aspect provides the advantageous discussed in association therewith.

Embodiments of the present invention will now be described by way of non-limitative example with reference to the accompanying drawings, in which:

Fig. 1 is a flowchart of a method of training a machine-learning algorithm to determine a sleep state;

Fig. 2a) and Fig. 2b) illustrate how to calculate the angle between a camera and the vertical;

Fig. 3a) to Fig. 3c) show how a homography transformation can be applied to obtain a virtual viewpoint looking directly downward;

Fig. 4a) to Fig. 4d) illustrate the calculation of an optical flow field;

Fig. 5a) and Fig. 5b) show a measure of subject movement derived from optical flow and an accelerometer signal, and the cross-correlation therebetween;

Fig. 6 is a flowchart of a method of determining a sleep state of a subject using a trained machine-learning algorithm;

Fig. 7 is a flowchart of a method of determining a sleep state of a subject using a machine-learning algorithm;

Fig. 8 shows classification of sleep state into wake, light sleep, or deep sleep by a machine-learning algorithm;

Fig. 9 shows a continuous measure of sleep depth of a subject determined by a machine-learning algorithm;

Fig. 10 shows classification of sleep state into wake, light sleep, medium sleep, or deep sleep by a machine-learning algorithm, and a corresponding continuous measure of sleep depth;

Fig. 11 is a flowchart of a method of determining a sleep state as wake, light NREM sleep, REM sleep, or deep NREM sleep using a cascaded machine-learning algorithm;

Fig. 12 shows further classification of sleep states classified as light sleep into light NREM or REM sleep;

Fig. 13 shows classification of sleep state into wake, light NREM sleep, REM sleep, and deep NREM sleep using a combination of three-state and two-state classification;

Fig. 14 shows classification of sleep state into wake, light NREM sleep, REM sleep, medium NREM sleep, or deep NREM sleep by a machine-learning algorithm using a combination of four-state and two-state classification, and a corresponding continuous measure of sleep depth;
Fig. 15 shows sleep depth pseudo-hypnograms generated for each of seven subjects;
Fig. 16 shows sleep depth pseudo-hypnograms using an additional medium sleep training label; and
Fig. 17 shows results from a two-stage classification algorithm.
Fig. 18a) to Fig. 18d) shows confusion matrices from a single-stage classifier that determines a sleep state of a subject.

The current application relates to methods of training a supervised machine-learning algorithm to determine a sleep state of a subject, and methods of determining a sleep state of a subject using machine-learning algorithms.

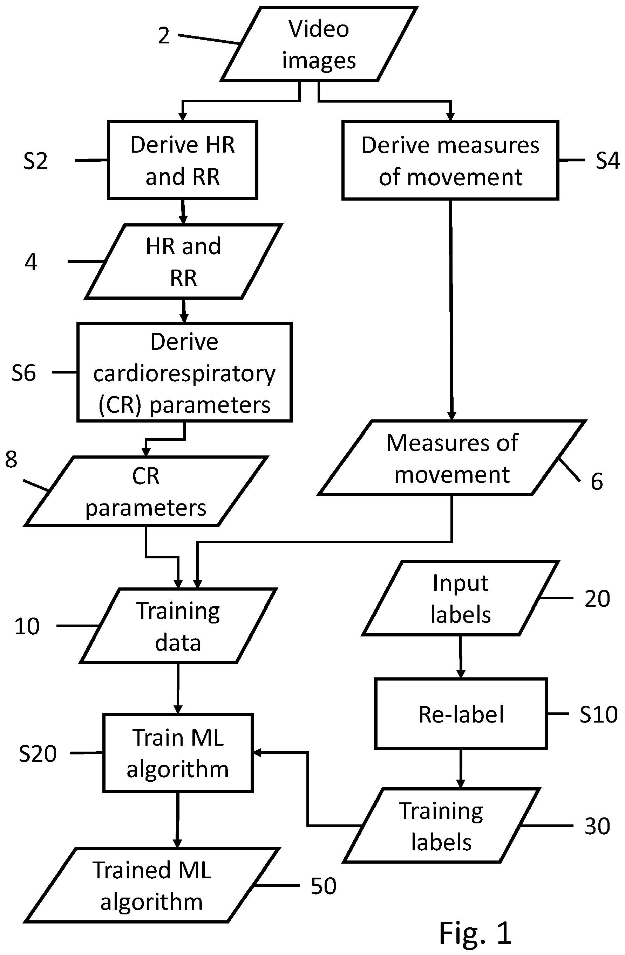

Fig. 1 is a flowchart showing a method of training a machine-learning algorithm to determine a sleep state of a subject. The method uses training data 10 in respect of a plurality of subjects derived from video images 2 of the subjects.

The video images 2 can be captured using a conventional visible-light camera, such as may be used for video surveillance. Vital sign characteristics can be extracted from the video images by detecting signals from changes in colour of a subject's skin and/or movements of the subject's body. These signals are indicative of changes in blood flow and movements of the chest during breathing. Various methods for extracting respiratory rates and heart rates from video images of a subject are known in the art and may be used in the present methods. An example is the Oxevision system provided by Oxehealth Ltd, and the techniques discussed in European patent application No. 19220087.1.

The training data 10 in respect of each subject are temporally divided into a plurality of epochs. These epochs are generally of equal length, typically 30 seconds, although this is not essential and other lengths of epoch may be used.

The training data comprises at least one feature derived from the video images of the subjects. The features may be derived directly from the video images, or may be derived from time-resolved measurements that are themselves derived from the video images, such as respiratory rates and heart rates. The at least one feature comprises at least one measure of subject movement 6 and at least one cardiorespiratory parameter 8 of the subject.

As shown in Fig. 1, the method comprises deriving the training data 10 from the video images 2 of the subjects. The training data 10 may be derived in various ways as discussed below.

In particular, the training data 10 may be fully or partially derived using a machine-learning algorithm. In this situation, the deriving of the training data 10 and the determining of a sleep state of the subject may be carried out by separate machine-learning algorithms. Alternatively, the deriving of the training data 10 and the determining of a sleep state of the subject may be performed together by a combined machine-learning algorithm, such as a deep neural network. In the discussion of determining sleep state that follows, references to the 'machine-learning algorithm' will generally refer (unless identified otherwise explicitly or by context) to the machine learning algorithm or part of the combined machine-learning algorithm that determines the sleep state of the subject. For example, in the case of a combined machine-learning algorithm in the form of a deep neural network, this could refer to the last stage classification layer.

Deriving the training data 10 comprises deriving S4 the measures of subject movement 6 from the video images 2. The measures of subject movement 6 may comprise a measure of subject movement in a head region of the subject and/or a measure of movement in a torso region of the subject.

The measure of the movement 6 in a head region of the subject is derived S4 from the video images 2 by segmenting a region of the video frame that is known or likely to contain the subject's head, for example the head region of the bed in which the subject is sleeping. The head region may be segmented from a rectified projection of a reference image frame. The measure of movement 6 in a torso region of the subject can be derived S4 using the same process, but with the image segmented so as to cover a region over the region of the image known or likely to contain the subject's torso.

In a clinical setting bed and camera positions are likely to vary, therefore it is important that any system-in-use is invariant to changes in either of these parameters. An image homography can be used to improve the robustness of the method to the camera angle.

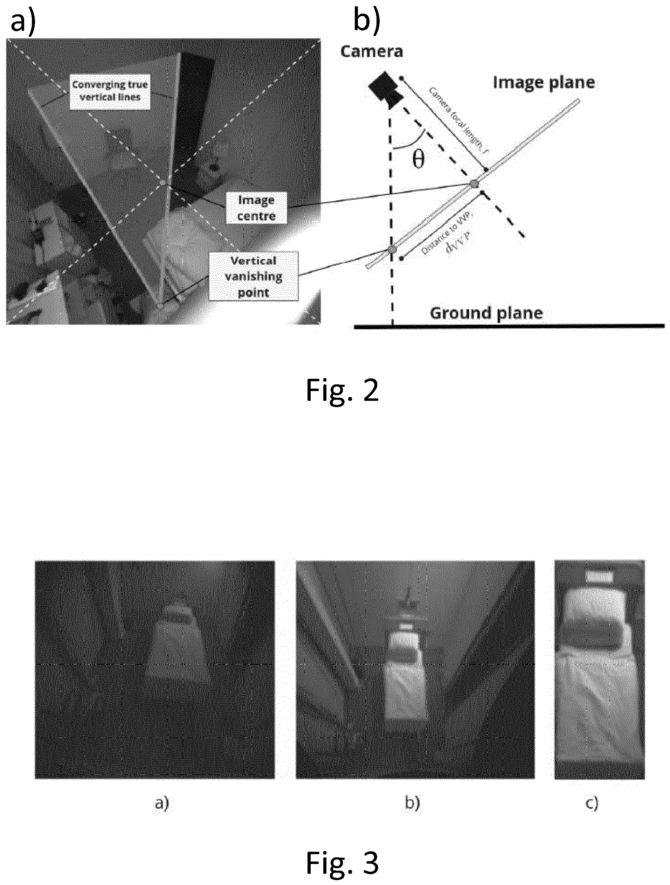

Using a still image and knowledge of the video camera parameters including its focal length it is possible to calculate the angle between the camera and the vertical. This process is illustrated in Fig. 2. The vertical vanishing point and camera angle from the vertical can be calculated. The vertical vanishing point (VVP) is calculated by finding the convergence point of true vertical lines in the image such as wall edges. The focal length and distance to the VVP in the image plane can be determined from the camera parameters and pixel coordinates. This allows the angle between the camera and the vertical $\theta$, to be calculated using simple trigonometry.

Using the camera angle, a homography transformation is applied to obtain a virtual viewpoint looking directly downward. A rotation about the vertical axis is also applied such that the bed is aligned to the vertical. After applying the transformation the images can be cropped to the bed region for further video processing. This process is illustrated in Fig. 3. Camera angle invariance is achieved using image rectification. Fig. 3a) shows an example frame. In Fig. 3b), the frame is rectified using a homography transformation. In Fig. 3c) the frame is cropped to the bed region for further processing

To derive S4 the measure of subject movement 6, an image optical flow signal is computed at a suitable frequency (for example 1-10 Hz, preferably 3-6 Hz, most preferably 5 Hz) over the segmented head region. Optical flow is the apparent motion of pixels in the image plane between frames. Sparse optical flow methods track the movement of a small number of points in the images over time, while dense optical flow methods compute a vector field over the entire image which indicate the movement of each pixel between frames.

From the cropped and rectified view of Fig. 3, the dense optical flow field over the bed region can be calculated between frames up to the frame rate of the camera (e.g. 20 FPS). The algorithm used here for calculating dense optical flow field is the Dense Inverse Search algorithm, because it has low computational complexity and high accuracy, but in general any suitable algorithm can be used.

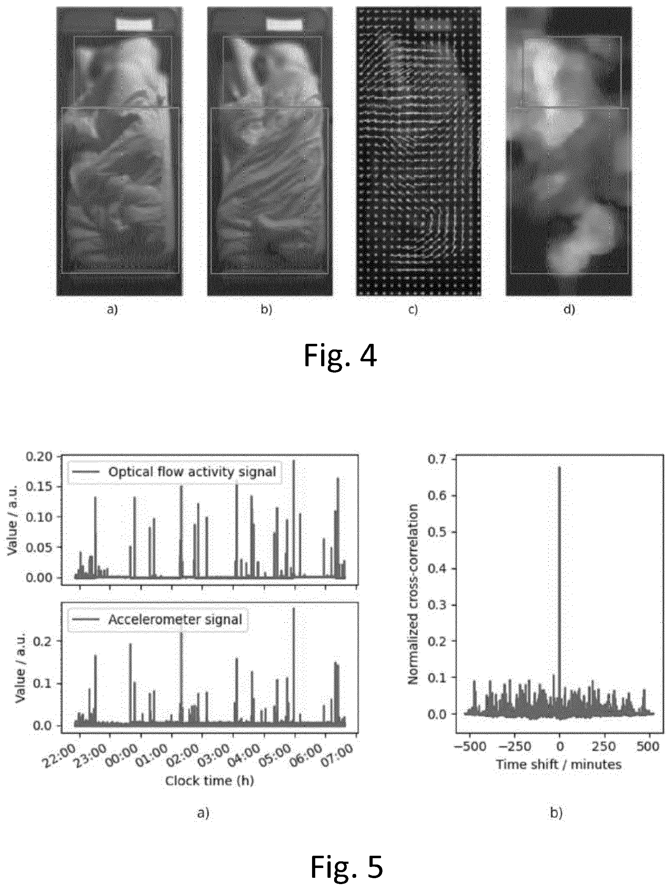

Fig. 4 shows an example optical flow field captured during a small change in body position during sleep. Fig. 4a) and Fig. 4b) show two frames captured one second apart. Fig. 4c) shows the resulting optical flow field and Fig. 4d) shows the corresponding magnitude image. The participant's face has been blurred.

From the optical flow field it is possible to calculate a continuous time series measure of subject movement 6. A simple method for doing this is to use the average magnitude of the flow field. This idea can be extended by calculating separate measures for the head and torso regions as mentioned above. This retains some information about the spatial extent of movement. Both of these measures are calculated at a suitable frequency, e.g. 5Hz. The frequency is chosen as a trade-off between temporal resolution and computation time. The head region (red) and torso region (blue) bounding boxes, used to derive the corresponding measures of subject movement are plotted in Fig. 4. The bounding boxes in Fig. 4 are manually specified, but the bounding boxes may also be automatically identified, for example using deep learning approaches.

Fig. 5a) shows a measure of subject movement 6 derived from optical flow, and also shows an accelerometer signal recorded at the same time as the video image from which the optical flow is determined. The source of the accelerometer signal is a PSG recording device attached to the patient's chest. The optical flow signal uses the average magnitude of the flow field in the torso region. Fig. 5b) shows the normalised cross-correlation between the two signals. Cross-correlation measures the similarity between two time series as a function of the relative shift between them. Fig. 5b) shows a sharp peak in the cross-correlation for zero time shift, indicating there is good correlation between the two signals.

The magnitude of the optical flow signal is processed by subtracting the mean value for each recording, and filtering the subtracted signal using a Gaussian smoothing filter (e.g. with $\sigma = 600$ seconds). The resulting filtered signal may then be downsampled by interpolation at a lower frequency (e.g. less than 5 Hz, preferably 1 Hz). Further normalisation may be performed by a suitable reference value, for example the 90th percentile value for that recording. For a given epoch, the measure of the movement in the head region of the subject was calculated as an average (e.g. the mean) of the values of the measure in a time window centred on that epoch. The time window may have any suitable length, for example 10 minutes. This particular process of extracting the measure is not essential, however, and any other suitable process may be used that results in a quantity representative of the subject's movement in a head region.

For both measures of movement 6, values may still be returned even where the subject is not in the bed, and therefore not within the segmented regions corresponding to the head region and torso region. To avoid distorting the training of the machine-learning algorithm, a further feature may be included indicating a binary value of whether the subject is considered to be in bed. This could be determined by conventional image recognition techniques used to detect the presence of a subject in the corresponding areas of the video image.

For either measure of movement 6, pre-processing may be used to reduce or remove drift in the baseline signal (also known as baseline wander), and reduce or remove spurious peaks. The signal may also be calibrated based on an initial wake period of the recording of the subject's sleep opportunity, i.e. the period before the subject has initially fallen asleep.

Deriving the training data 10 further comprises deriving S2 heart rate (HR) and respiratory rate (RR) 4 from the video images 2. The cardiorespiratory parameters 8 are then derived S6 from the HR and RR 4.

As mentioned above, the respiratory rate (RR, also referred to as breathing rate or respiration rate) can be extracted from the video images 2 by various known techniques. Respiration causes movements of the chest wall which can be measured to calculate a rate of respiration through methods such as frame-differencing, using dense optical flow magnitudes or by tracking the movement of sparse optical flow vectors in the video sequence.

To improve the consistency of the data and reduce the impact of spurious measurements, it is preferable that the RR for each epoch is calculated as an average (e.g. the mean) of the RR values in a time window centred on that epoch. The time window may have any suitable length, for example 10 minutes. Since this averaging is performed, the respiratory rates used may be those before any filtering is performed by the process used to extract the RR from the video images.

The heart rate (HR) can also be derived from the video images 2 by various known techniques. Pulses of blood caused by heart beats cause fluctuations in the colour of a subject's skin, caused by the variation in blood oxygenation over the cardiac cycle and often referred to as "micro-blushes", and which can be detected by a camera. Heart rate can also be detected from head and body movements, caused by the ballistic forces generated as the heart pumps blood through the aorta. The measurement of these movements is known as ballistocardiography.

To improve the consistency of the data and reduce the impact of spurious measurements, it is preferable that the HR for each epoch is calculated as an average (e.g. the mean) of the HR values in a time window centred on that epoch. The time window may have any suitable length, for example 10 minutes. Since this averaging is performed, the heart rates used may be those before any filtering is performed by the process used to extract the HR from the video images.

The cardiorespiratory parameters 8 may be human recognisable or feature engineered quantities that are selected because they are known to contain relevant information content about the sleep state of a subject. Examples of such quantities comprise a respiratory rate of the subject, a variance of the respiratory rate of the subject, a heart rate of the subject, and/or a variance of the heart rate of the subject.

The variance of the respiratory rate (RRV) may be calculated as the standard deviation of the RR values extracted from the video images 2. As for the RR, the RRV for each epoch may be calculated using the RR values in a time window centred on that epoch. The time window may have any suitable length, for example 10 minutes. The variance of the heart rate (HRV) may be calculated as for the RRV.

The cardiorespiratory parameters 8 may alternatively or additionally comprise one or more machine-learnt features that are do not necessarily correspond to human-recognisable quantities. These can be derived using a neural network. In this case, the deriving S6 of the cardiorespiratory parameters 8 comprises using a neural network. The cardiorespiratory parameters 8 would then comprise one or more of the "deep" features from the hidden layer or reduced representation layer of the neural network. The neural network used to derive S6 the cardiorespiratory parameters 8 (that make up part of the training data 10) is separate from the machine learning algorithm discussed in more detail below which is trained using the training data 10 to determine a sleep state of a subject (and which may also be neural network). The neural network used to derive the cardiorespiratory parameters 8 may be trained in any suitable manner. For example, it may be trained by classifying sleep states based on only the HR and RR values extracted from the video images 2. The resulting cardiorespiratory parameters 8 would then be combined with the measures of subject movement 6 to form the training data 10 that is used to train the supervised machine learning algorithm as described further below.

For any of the input features, the values used to calculate the overall feature value for an epoch may also be weighted using a confidence metric. In some systems used to extract the training data 10 from video images, a value may be provided for a confidence in each value. The values may then be weighted by the confidence value when calculating an overall average for the epoch, and/or may be excluded entirely if the confidence is considered too low (i.e. falls below a predetermined threshold such as 50% confidence). This can help to reduce the impact of spurious signals.

As well as the values for the various quantities derived from the video images 2 and making up the training data 10 discussed above, each epoch in the training data 10 is associated with a respective input label 20 indicating that a sleep state of the epoch is one of a set of sleep states comprising wake, rapid-eye movement (REM) sleep, light non-rapid eye movement (NREM) sleep, or deep NREM sleep. These sleep states may also be referred to using their designations under the commonly-used Rechtschaffen and Kales (R&K) sleep scoring system used by the American Academy of Sleep Medicine (AASM), which are W, REM, N1, and N3 respectively.

In what follows, epochs associated with an input label 20 indicating that a sleep state of the epoch is wake may be referred to as wake epochs, epochs associated with an input label 20 indicating that a sleep state of the epoch is light NREM sleep may be referred to as light NREM epochs, and so on for the other input labels.

The input labels 20 will typically be obtained from interpretation by a trained polysomnography scorer of the training data and/or other data collected at the same time as the video images from which the training data are derived (for example from sensors worn by the subject). This establishes definitions of the 'true' sleep state for each epoch against which the machine-learning algorithm can be trained.

The method comprises training S20 the supervised machine learning algorithm using the training data and training labels. A training label 30 is associated with each epoch in the training data 10.

The training label 30 is selected from a set of training labels 30 comprising training labels 30 indicating that the sleep state of the epoch is wake, light sleep, or deep sleep.

In general, there may be epochs in the training data 10 that do not have an associated training label 30, for example because they do not have an input label 20, or because they are excluded for other reasons. For example, epochs missing values for one or more of the quantities discussed above may be excluded. Such epochs would not be used to train the machine-learning algorithm.

The distinction between the input labels 20 that are provided with the training data 10, and the training labels 30 that are used to train the machine-learning algorithm makes it possible to simplify the determination of the sleep state to improve its reliability as discussed above. This can be achieved through a processing of relabelling S 10 the input labels 20 to produce the training labels 30.

In step S10, epochs associated with an input label 20 indicating that the sleep state of the epoch is REM sleep or light NREM sleep are associated with a training label 30 indicating that the sleep state of the epoch is light sleep. By combining REM sleep epochs with light NREM sleep epochs, the machine-learning algorithm does not have to distinguish between REM and NREM sleep states, which can greatly improve the reliability of determination of the sleep state.

In step S 10, epochs associated with an input label 20 indicating that the sleep state of the epoch is wake are associated with a training label 30 indicating that the sleep state of the epoch is wake, and epochs associated with an input label indicating that the sleep state of the epoch is deep NREM sleep are associated with a training label 30 indicating that the sleep state of the epoch is deep sleep.

In general, epochs associated with a particular training label 30 may be referred to as being epochs 'in' the class of that training label 30.

The set of sleep states indicated by the input labels 20 may further comprise intermediate NREM sleep. Polysomnography studies typically label an intermediate NREM sleep state in addition to the light and deep NREM sleep states. Intermediate (or medium) NREM sleep may be referred to as N2 sleep under the R&K system used by the AASM. Intermediate NREM sleep can be difficult to distinguish from light and deep NREM sleep, because of its similarities to both other sleep depths. Nonetheless, these intermediate NREM sleep epochs can provide valuable additional data for training the machine-learning algorithm.

One possibility for making use of the intermediate NREM epochs is to include them in one of the other training labels 30. In this case, in the relabelling step S10, a proportion of the epochs associated with an input label 20 indicating that the sleep state of the epoch is intermediate NREM sleep are associated with a training label 30 indicating that the sleep state of the epoch is light sleep. This approach extends the number of light NREM sleep epochs available for training.

The intermediate NREM sleep epochs associated with the light sleep training label 30 are preferably those most closely resembling light NREM sleep. To achieve this, the proportion of the epochs associated with an input label 20 indicating that the sleep state of the epoch is intermediate NREM sleep may comprise epochs associated with an input label 20 indicating that the sleep state of the epoch is intermediate NREM sleep that immediately precede or follow an epoch associated with an input label 20 indicating that the sleep state of the epoch is light NREM sleep.

In some of the results discussed below, the training label 30 indicating that the sleep state of the epoch is light sleep may be referred to as a 'light' class where no intermediate NREM sleep epochs are associated with the light sleep training label, and as a 'light*' class when intermediate NREM sleep epochs are associated with the light sleep training label 30.

The intermediate NREM sleep epochs immediately preceding or following a light NREM sleep epoch may include a plurality of consecutive intermediate NREM sleep epochs preceding or following the light NREM sleep epoch. For example, four or eight consecutive intermediate NREM sleep epochs preceding or following a light NREM sleep epoch may be included in the proportion of intermediate NREM sleep epochs. Eight intermediate NREM epochs were used in the results discussed below. The remaining intermediate NREM sleep epochs may be excluded from use in the training of the machine-learning algorithm.

Another option for making use of the intermediate NREM epochs is to increase the number of training labels 30, such that the set of training labels 30 further comprises a training label 30 indicating that the sleep state of the epoch is medium sleep. Epochs associated with an input label 20 indicating that the sleep state of the epoch is intermediate NREM sleep are associated with the training label 30 indicating that the sleep state of the epoch is medium sleep. In this case, the intermediate NREM epochs can also contribute to determining the sleep state.

The machine-learning algorithm may be trained and tested using any suitable method, for example leave-one-out cross-validation on a per-night basis. Various different types of machine-learning algorithm may be used for the determination of the sleep state, depending on how the determined sleep state is to be used.

The machine learning algorithm may be a regression algorithm, for example a neural network. An example of a suitable neural network for the regression algorithm is a multilayer perceptron regressor. In this case, the machine-learning algorithm determines the sleep state using a continuous measure of sleep depth. The continuous measure is preferably normalised so that it can be easily compared between different subjects.

In the example above, three training labels 30 are used corresponding to the sleep state being wake, light sleep, or deep sleep. In this case, the continuous measure may be a number between -1 and +1, where a value of +1 represents wake, a value of 0 represents light sleep, and a value of -1 represents deep sleep. The particular range of values chosen as the scale for sleep depth is not particularly limited. For example, in other embodiments, a value of 0 may represent wake and a value of 1 may represent deep sleep.

The machine learning algorithm may be a classification algorithm, for example one of a logistic regression, k-nearest neighbours, a random forest classifier, and a neural network. An example of a suitable neural network is a neural multilayer perceptron. A random forest classifier is less prone to over fitting than a neural network in the low data regime, so may be preferred in such situations and may allow more features from the training data 10 to be used simultaneously without observing strong overfitting. A Random Forest is also better able to handle missing data, so may be preferred where data are incomplete. In this case, the machine-learning algorithm may determine the sleep state to be one of wake, light sleep, or deep sleep. Alternatively, where the set of sleep states further comprises intermediate NREM sleep and the set of training labels 30 further comprises a training label 30 indicating that the sleep state of the epoch is medium sleep, the supervised machine-learning algorithm may determine the sleep state to be one of wake, light sleep, medium sleep, or deep sleep.

Where the machine-learning algorithm is a classification algorithm, it may still output a numerical value (or score) for each of the training labels indicating how the sleep state is to be classified. In such embodiments, the classification algorithm generates scores for each of the training labels representing a confidence of the sleep state being the sleep state indicated by the training label.

The ultimate determination of the sleep state as a classification into one of the sleep states indicated by the training labels may be determined from these scores in various ways, for example by choosing the sleep state associated with the training label having the highest classification score. However, the scores themselves can also be used to calculate measures of the sleep depth. For example, the supervised machine-learning algorithm may determine the sleep state using a continuous measure of sleep depth, where the continuous measure is determined from a weighted combination of the scores for each of the training labels.

In the examples discussed above, the set of training labels comprises three or four labels, representing wake, light sleep, deep sleep, and optionally medium sleep. Where there are three training labels, the classification algorithm is trained to determine the sleep state as a classification into one of three sleep states. It generates three scores: P(W), P(L) and P(D), which are the classification score for wake, light sleep, and deep sleep respectively. To aid in interpretability and comparison between subjects, the scores are preferably normalised such that the sum of the scores is equal to a predetermined value, e.g. 1.0.

The scores can then be combined into a continuous measure of sleep depth. A typical relationship used to combine the scores is as follows:

$$\text{measure of sleep depth} = 0*P(W) + 0.5*P(L) + 1*P(D)$$

Using this relation, and where the scores are normalised to sum to 1, wake results in a value of 0, light sleep results in a value of 0.5, and deep sleep results in a value of 1.

Where there are four training labels including medium sleep, the classification algorithm is trained to determine the sleep state as a classification into one of four sleep states. It generates four scores: P(W), P(L), P(M) and P(D), which are the classification score for wake, light sleep, medium sleep, and deep sleep respectively. As before, these can be normalised to aid interpretability. A typical relationship used to combine the scores in the four class case is as follows:

$$\text{measure of sleep depth} = 0*P(W) + \tfrac{1}{3}*P(L) + \tfrac{2}{3}*P(M) + 1*P(D)$$

The particular values for the relations shown here are of course not essential, and different values may be chosen depending on factors such as the desired range of values for the measure of the sleep state.

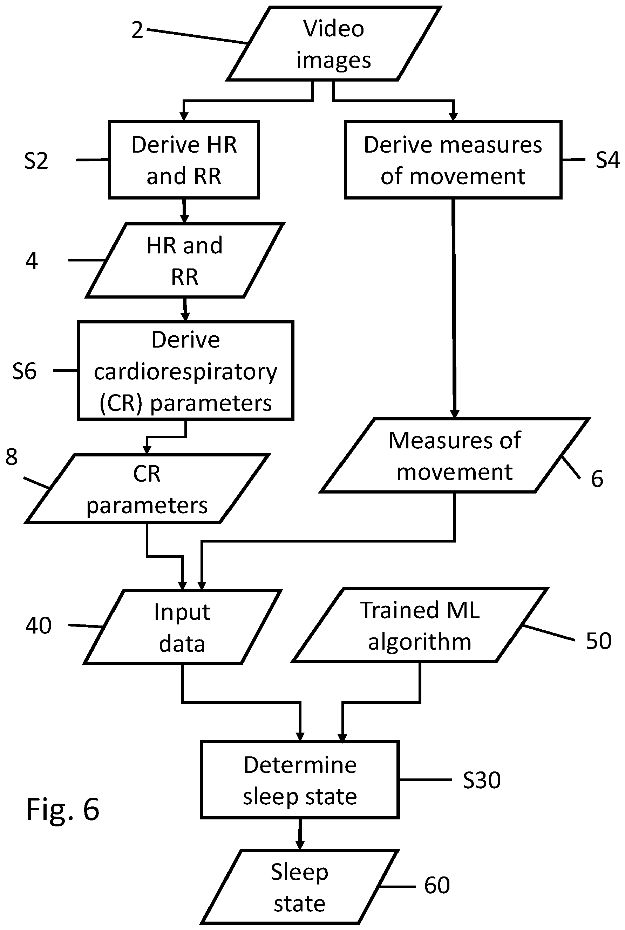

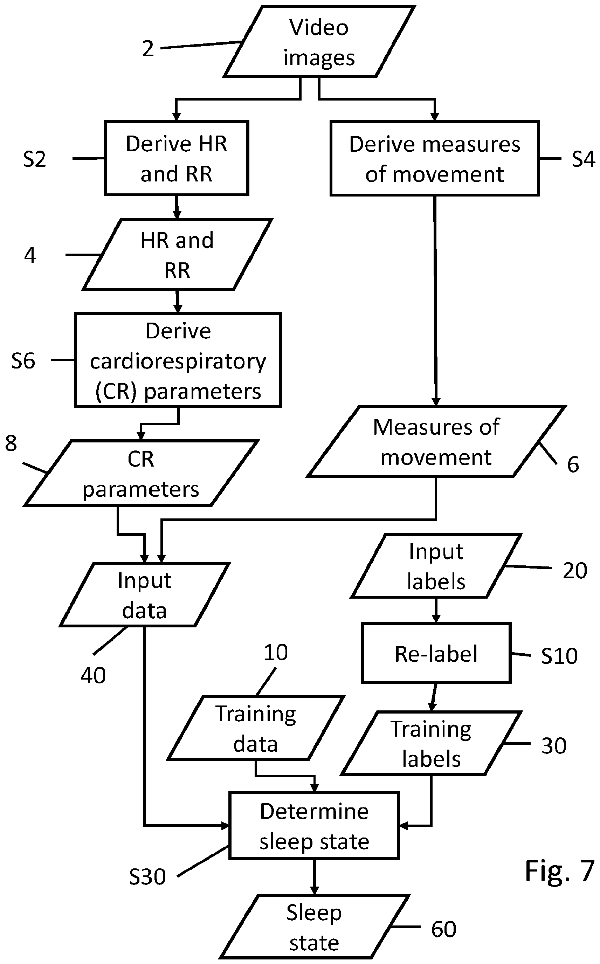

Corresponding to the methods of training a supervised machine-learning algorithm discussed above, there are also provided methods of determining a sleep state 60 of a test subject as shown in Figs. 6 and 7. The method comprises applying a machine-learning algorithm to input data 40 from the test subject. The supervised machine-learning algorithm outputs a determination of the sleep state 60 of the test subject on the basis of training data 10 and training labels 30. As for the training methods described above, the input data 40 are derived from video images 2 of the test subject. The video images 2 can be analysed in the same way as described above for the training data 10 to obtain the input data 40 for the test subject. Also as discussed above, the input data 40 may be fully or partially derived using a machine-learning algorithm. The deriving of the input data 40 and the determining of a sleep state of the subject may be carried out by separate machine-learning algorithms or by a combined machine-learning algorithm, such as a deep neural network. In the discussion that follows, references to the 'machine-learning algorithm' will generally refer (unless identified otherwise explicitly or by context) to the machine learning algorithm or part of the combined machine-learning algorithm that determines the sleep state of the subject.

Specifically, the measures of movement 6 are derived S4 from the video images 2 of the test subject. Heart rate and respiration rate 4 are derived S2 from the video images 2. The cardiorespiratory parameters 8 are derived from the HR and RR 4. The input data 40 are then composed of the derived cardiorespiratory parameters 8 and measures of movement 6 for the test subject.

The training data 10 is in respect of a plurality of training subjects and is derived from video images 2 of the training subjects, as described above. The training data 10 and training labels 30 are otherwise as described above in relation to the training methods. There are two main option for the determination of the sleep state 60 of the test subject on the basis of the training data 10 and training labels 30. Which of these is used will depend on the type of machine-learning algorithm that is being used.

The first option is shown in Fig. 6, where the machine-learning algorithm is trained using a method of training a supervised machine learning algorithm, as discussed above. In this case, the machine-learning algorithm is pre-trained, and the input data 40 and trained machine-learning (ML) algorithm 50 are used together to determine S30 the sleep state 60. The input to step S30 corresponding to the trained ML algorithm 50 may comprise a plurality of parameters that were determined by the method of training the machine-learning algorithm. The input data 40 are derived from the video images 2 of the test subject using the steps described above in connection with Fig. 1. Deriving the input data 40 comprises deriving S2 heart rate and respiratory rate 4 from the video images of the test subject and deriving S6 the cardiorespiratory parameters 8 for the test subject from the heart rate and respiratory rate 4. Deriving the input data 40 further comprises deriving S4 the measures of movement 6 from the video images 2 of the test subject. The input data 40 are then fed into the trained machine-learning algorithm 50, which outputs the determination of the sleep state 60. Neural networks such as the multi-layer perceptron (whether as a regressor or classifier) are an example of this type of machine-learning algorithm.

The second option is shown in Fig. 7, where the machine-learning algorithm is of a type which is not pre-trained, but compares the input data 40 to the training data 10 at the time of being run. Again, the video images 2 of the test subject are used to derive the input data 40 as described above. In this case, the relabelling S10 of the epochs in the training data 10 is carried out during the method, and the training data 10 are required to determine the sleep state for subsequent new input data 40 from test subjects. The training data 10 may also be derived from the video images 2 of the training subjects at the time of carrying out the method using the steps described above in connection with Fig. 1. However, preferably, and as shown in Fig. 7, the training data 10 are derived from the video images 2 of the training subjects at an earlier time and provided at the time of carrying out the method. Since the training data 10 do not change for different test subjects, this reduces computational requirements by reducing the amount of processing that must be carried out for each new test subject.

Regardless of which option is used, the determination of the sleep state of the test subject may be a value of a continuous measure of sleep depth, or a classification of the sleep state, depending on the type of machine-learning algorithm used. The classification may be as one of wake, light sleep, or deep sleep. In some embodiments where the set of sleep states further comprises intermediate NREM sleep, the set of training labels may further comprise a training label indicating that the sleep state of the epoch is medium sleep and the determination of the sleep state of the test subject may be a classification of the sleep state as one of wake, light sleep, medium sleep, or deep sleep. Where the machine-learning algorithm is pre-trained, it may be trained using any suitable corresponding method described above.

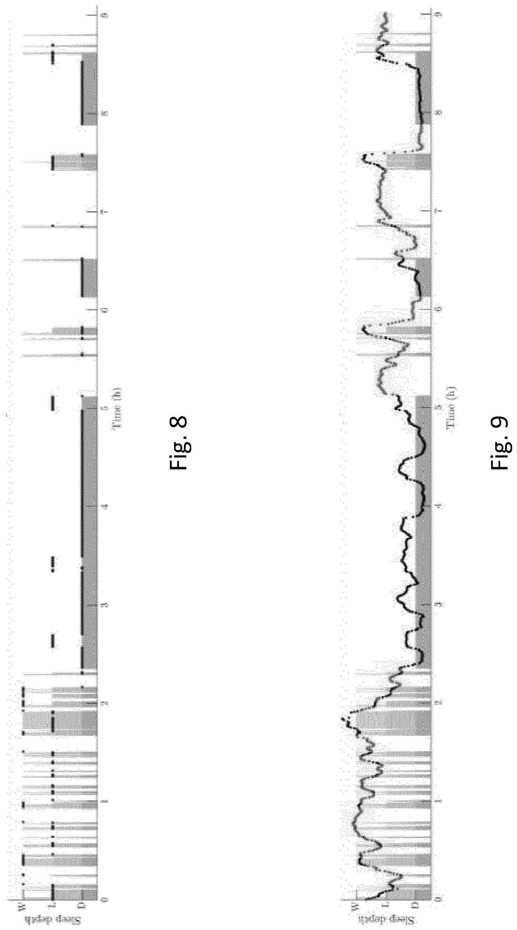

Fig. 8 shows the results of an example where the machine-learning algorithm is a classification algorithm and the determination of the sleep state is a classification of the sleep state as one of wake, light sleep, or deep sleep. In this example, the training data 10 and input data 40 comprise the respiratory rate (RR), the respiratory rate variance (RRV), the measure of movement in the head region, and the measure of movement in the body region from video image analysis. The machine-learning algorithm is a multilayer perceptron. The machine-learning algorithm has been applied to the determination of the sleep state for a 10-hour sleep opportunity. The classifications of each epoch from the machine-learning algorithm as wake, light sleep, and deep sleep are shown in black. A classification of each epoch as wake, light sleep (N1 or REM) and deep sleep (N3) based on the input labels 20 from the polysomnography scorer are shown as grey-shaded areas. Epochs having an input label 20 of N2 (intermediate NREM sleep) are omitted. As can be seen, the classifications from the machine-learning algorithm closely follow those from the polysomnography scorer.

Fig. 9 shows the results of an example where the machine-learning algorithm is a regression algorithm, and the determination of the sleep state is a value of a continuous measure of sleep depth. The regression algorithm uses the same input data 10 as shown in Fig. 8, and the machine-learning algorithm is again a multilayer perceptron. The classification of each epoch as wake, light sleep (N1 or REM) and deep sleep (N3) based on the input labels 20 from the polysomnography scorer are again shown as grey-shaded areas. While there is more variability in the continuous measure, it can still be seen that the continuous measure tracks the classifications from the polysomnography scorer. The values output by the regression algorithm for epochs associated with an input label 20 of wake, N1, N3 and REM are shown in black, while the values for epochs associated with an input label 20 of N2 are shown in red.

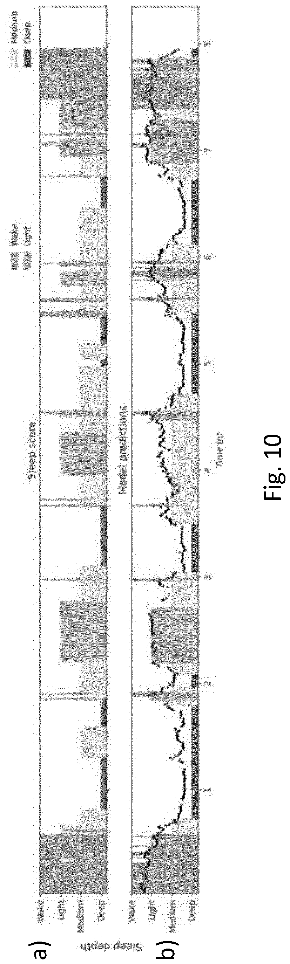

Fig. 10 shows results for the case where the set of sleep states further comprises intermediate NREM sleep, and the set of training labels 30 further comprises a training label indicating that the sleep state of the epoch is medium sleep. The results are shown for a sample recording from a video image over an 8 hour period. The machine-learning algorithm is a classification algorithm. Fig. 10a) shows the classification of each epoch as wake, light sleep (N1 or REM), medium sleep (N2), and deep sleep (N3) based on the input labels 20 from the human polysomnography scorer. Fig. 10b) shows the determination of the sleep state for each epoch by the machine-learning algorithm as a classification of the sleep state as one of wake, light sleep, medium sleep, and deep sleep using coloured vertical blocks. Fig. 10b) also shows in black the determination of the sleep state for each epoch by the machine-learning algorithm as a continuous measure of sleep depth. The continuous measure is determined from a weighted combination of scores generated by the classification algorithm for each of the training labels, using the formula described above. As can be seen, both the classification and the continuous measure from the machine-learning algorithm track closely the classifications from the human scorer.

While the methods above improve the reliability and accuracy of the determination of the sleep state in terms of its depth, it is also desirable to be able to separate REM sleep, which is an important stage of the sleep cycle. This can be performed by a machine-learning algorithm analogous to those described above, but separating NREM and REM sleep.

The machine-learning algorithm may be trained using a method of training a supervised machine-learning algorithm to determine a sleep state of a subject, which is also described by the flowchart in Fig. 1. The method uses training data 10 in respect of a plurality of subjects derived from video images of the subjects. The training data 10 is as described above. Each epoch of the training data 10 is associated with a respective input label 20 indicating that a sleep state of the epoch is one of a set of sleep states comprising rapid-eye movement (REM) sleep and light non-rapid eye movement (NREM) sleep.

The method comprises training the supervised machine learning algorithm using the training data 10 and a training label 30 associated with each epoch. The training labels 30 are analogous to those described above, except that the set of training labels 30 comprises training labels 30 indicating that the sleep state of the epoch is NREM sleep or REM sleep.

In step S10, epochs associated with an input label 10 indicating that the sleep state of the epoch is REM sleep are associated with a training label 30 indicating that the sleep state of the epoch is REM sleep. The machine-learning algorithm may then separate NREM and REM sleep in different ways. There are three main alternatives for doing this, which differ in what epochs from the training data 10 are associated with the NREM training label. The alternatives are:

1. Use epochs associated with the input label 20 of light NREM sleep (N1) as the non-REM class. In this case, epochs associated with an input label 20 indicating that the sleep state of the epoch is light NREM sleep are associated with a training label indicating that the sleep state of the epoch is NREM sleep;

2. Use epochs associated with any input label 20 other than REM sleep i.e. (Wake, N1, N2 if used, and N3) as the non-REM class. In this case epochs associated with an input label 20 indicating that the sleep state of the epoch is a sleep state other than REM sleep are associated with a training label indicating that the sleep state of the epoch is NREM sleep; or

3. Use epochs associated with the N1 and Wake input labels 20 as the non-REM class. In this case, epochs associated with an input label 20 indicating that the sleep state of the epoch is wake or light NREM sleep are associated with a training label indicating that the sleep state of the epoch is NREM sleep. This may be preferred in some situations because the wake class is most different to REM sleep of the other NREM sleep states.

In each of these alternatives, the training data 10 may be artificially balanced by under-sampling of epochs associated with the input label 20 that occurs more frequently.

The machine-learning algorithm may then be trained to generate a score P(REM) representing a confidence that the sleep state of the epoch is REM sleep. As for the scores discussed for the depth of sleep above, the score may be normalised for interpretability, for example such that it falls between 0 (representing NREM sleep) and 1 (representing REM sleep).

Correspondingly, these principles above can be used to provide a method of determining a sleep state of a test subject, which can separate NREM and REM sleep. The method comprises applying a machine-learning algorithm to input data 40 from the test subject, wherein the input data are derived from video images of the test subject. The machine-learning algorithm outputs a determination of the sleep state of the test subject as light NREM sleep or REM sleep on the basis of the training data 10 and training labels 30. The machine-learning algorithm may operate substantially as described above, for example in terms of definition of the training labels 30 and the association of the epochs of the training data 10 with the training labels 30.

The machine-learning algorithm may use both the training data 10 and input data 40 at runtime, similarly as discussed in relation to Fig. 7. Alternatively, as discussed in relation to Fig. 6, the machine learning algorithm may be pre-trained and the method of determining a sleep state of the test subject comprises applying a machine-learning algorithm trained using a method as described above.

The classification of the sleep state in terms of sleep depth as wake, light sleep, or deep sleep (and optionally medium sleep) can be combined with the classification of the sleep state as NREM or REM in a cascade arrangement to fully characterise the sleep state. By separating the classification problem into two stages, the problem is simplified compared to a method that attempts to separate all of the sleep states from each other directly.

This cascade arrangement allows for the full classification of the sleep state into one of the five, commonly-used R&K states of wake, N1, N2, N3, and REM. The use of video images allows this to be done without the subjects having to wear obtrusive sensor equipment.

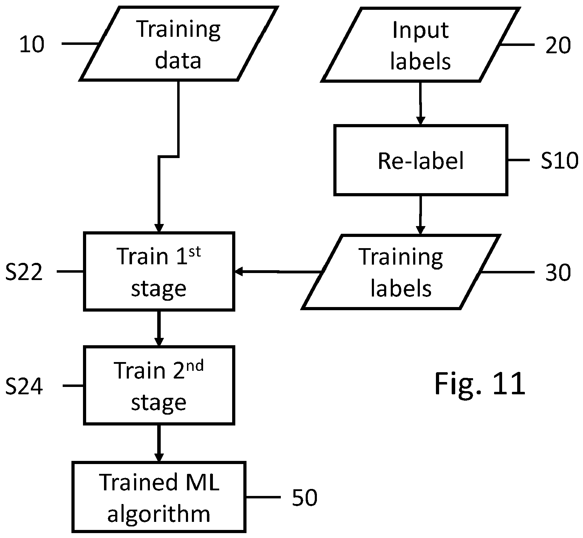

Fig. 11 shows a method of training a supervised machine-learning algorithm to determine a sleep state of a subject, wherein the machine-learning algorithm comprises a first-stage classification algorithm and a second-stage classification algorithm.

The method comprises training S22 the first-stage classification algorithm. This first stage can be performed using any of the methods of training described above (for example in relation to Figs. 8-10) where the machine-learning algorithm is a classification algorithm and the training labels 30 indicate the depth of sleep (i.e. that the sleep state is one of a set comprising wake, light sleep, deep sleep, and optionally medium sleep).

The method further comprises training S24 the second stage classification algorithm using the training data to further classify the sleep state as NREM sleep or REM sleep when the first stage classification algorithm classifies the sleep state as light sleep. This second stage can be performed using the methods of training described above where the set of training labels comprises training labels indicating that the sleep state of the epoch is NREM sleep or REM sleep. In the context of the cascade arrangement where the machine-learning algorithm has two stages, the training labels 30 used in further classifying the sleep state as NREM sleep or REM sleep may be referred to as second-stage training labels. However, the second-stage training labels are otherwise the same as described above, in particular with regard to the three options for which epochs are associated with the second-stage training label indicating that the sleep stage is NREM sleep.

The second-stage classification algorithm may be trained to generate a score P(REM) representing a confidence that the sleep state of the epoch is REM sleep. As for the scores in the first stage, the score may be normalised for interpretability, for example such that it falls between 0 (representing NREM sleep) and 1 (representing REM sleep). If the sleep state is determined to be light sleep, the second stage classification algorithm further classifies the light sleep as REM or NREM sleep. For example, when epochs have been classified as light sleep (L, N1 and REM) by the first stage classification algorithm, the second stage (REM) classification algorithm may be used to discriminate between REM/NREM sleep based on the score P(REM). Alternatively or additionally, an epoch may be classified as REM sleep by the second stage classification algorithm even where it was not initially classified as light sleep by the first stage classification algorithm. For example, where the score P(REM) exceeds a predetermined threshold, that epoch may be classified as REM sleep independently of its score according to the first stage classification algorithm.

Correspondingly, the principles above can be used to provide a method of determining a sleep state of a test subject, which is capable of more reliably separating REM sleep. The method comprises determining a sleep state of the test subject using one of the methods of determining the sleep state of the subject described above (for example in relation to Figs. 8-10) where the machine-learning algorithm is a classification algorithm and the training labels 30 indicate the depth of sleep (i.e. that the sleep state is one of a set comprising wake, light sleep, deep sleep, and optionally medium sleep). The method further comprises, if the sleep state is determined to be light sleep, further classifying the light sleep as REM or NREM sleep.

The further classification can be performed in various ways. Preferably, the further classification is carried out using a second stage machine-learning classification algorithm that outputs a classification of the sleep state as NREM sleep or REM sleep on the basis of the training data 10 and input labels 20. The second-stage classification algorithm may operate as described with respect to the training above.

The machine-learning algorithm may use both the training data 10 and input data 40 at runtime, similarly as discussed in relation to Fig. 7. Alternatively, as discussed in relation to Fig. 6, the machine learning algorithm may be pre-trained and the method of determining a sleep state of a test subject comprises applying a machine-learning algorithm trained using a method as described above, wherein the machine-learning algorithm comprises a first-stage classification algorithm and a second-stage classification algorithm. The machine-learning algorithm is applied to input data 40 from the test subject, the input data 40 derived from video images 2 of the test subject.

As a further alternative, the further classification of the sleep state as NREM sleep or REM sleep may use a simpler non-machine learning based approach. This could include a simple heuristic approach such as a threshold applied to one or more quantities in the input data 40. For example, where the input data comprise a respiratory rate of the test subject, the further classification may be carried out using the respiratory rate.

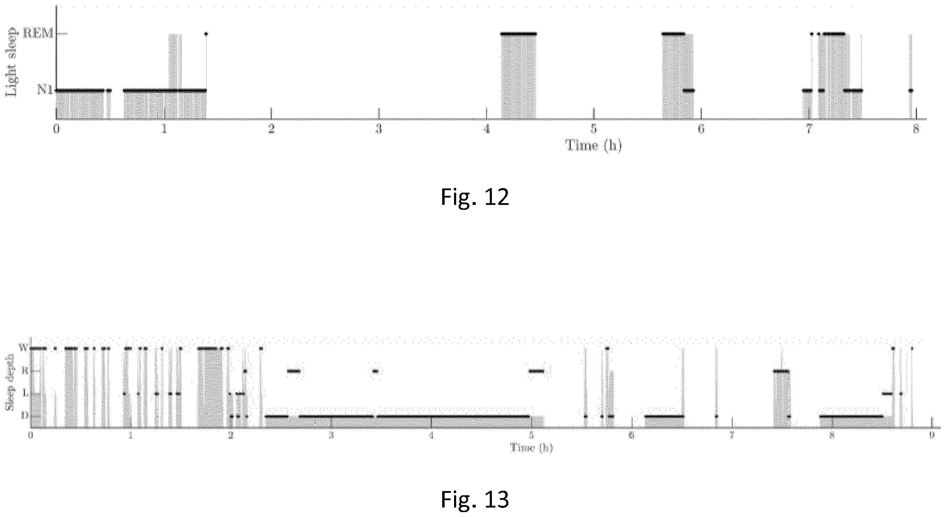

Fig. 12 shows results for a 10-hour sleep opportunity from the second-stage classification algorithm in an embodiment where the further classification is carried out using a second stage machine-learning classification algorithm. The output of the first classifier is used to trigger the use of the second classifier, such that epochs initially classified as light sleep are separated into light NREM sleep (N1) and REM sleep. In this example, the input data 40 comprise the respiratory rate, the respiratory rate variance (RRV), the measure of movement in the head region, and the measure of movement in the body region derived from video images of the test subject. N1 and REM scores from the second-stage classification algorithm are shown in black. The classification of each epoch as light NREM sleep and REM sleep based on the input labels 20 from the polysomnography scorer are shown as grey-shaded areas.

Fig. 13 shows a 'pseudo-hypnogram' generated for a 10-hour sleep opportunity using the full cascaded method of a first stage classification of epochs into wake/light sleep/deep sleep, followed by further classification of the light sleep epochs into light NREM and REM sleep. The classification determined by the machine-learning algorithm as wake, light NREM (N1), deep NREM (N3), and REM is shown in black. The classification of each epoch as wake, light NREM (N1), deep NREM (N3), and REM based on the input labels 20 from the polysomnography scorer are shown as grey-shaded areas. Classification results for epochs associated with an input label 20 of N2 (intermediate NREM sleep) are omitted. N2 epochs could be identified using regressor output values (intermediate values between those corresponding to N1 and those corresponding to N3).

The second-stage classification algorithm may generate a score P(REM) representing a confidence that the sleep state of the epoch is REM sleep, as discussed above. In this case, the second stage (REM) classification algorithm may be used to discriminate between REM/NREM sleep based on the score. Alternatively or additionally, an epoch may be classified as REM sleep by the second stage classification algorithm even where it was not initially classified as light sleep by the first stage classification algorithm. For example, where the score P(REM) exceeds a predetermined threshold, that epoch may be classified as REM sleep independently of its score according to the first stage classification algorithm.

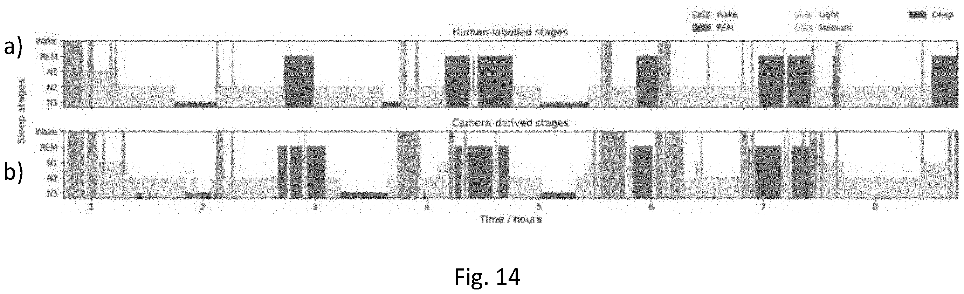

Fig. 14 shows results for two-stage classification algorithm in the case where the set of sleep states further comprises intermediate NREM sleep, and the set of training labels 30 further comprises a training label indicating that the sleep state of the epoch is medium sleep. The results are shown for a sample recording from a video image over an 8 hour period. The machine-learning algorithm is a classification algorithm. Fig. 14a) shows the classification of each epoch as wake, light NREM sleep, REM sleep, medium sleep (N2), and deep sleep (N3) based on the input labels 20 from the human polysomnography scorer. Fig. 14b) shows the determination of the sleep state for each epoch by the machine-learning algorithm as a classification of the sleep state as one of wake, light NREM sleep, REM sleep, medium sleep, and deep sleep using coloured vertical blocks. As can be seen, the classification by the machine learning algorithm tracks closely the classifications from the human scorer.

The following shows results from testing of embodiments of the methods discussed above to demonstrate their improved reliability.

Training data 10 were obtained from a dataset of normal, sleep-healthy volunteers. Normal sleep subjects (n = 6) were invited to sleep in a test room, where they underwent an overnight 10-hour sleep opportunity monitored with polysomnography (PSG).

PSG was conducted in accordance with the guidance of the American Academy of Sleep Medicine (AASM). Features were derived from physiological signals collected using contact-sensors (a SOMNOscreen from SOMNOmedics GmbH, Randersacker, Germany). All data were recorded using and stored off-line for scoring using standard AASM scoring criteria. Concurrent to the monitoring of sleep via PSG, video recording was obtained using a camera-based vital sign monitoring system (Oxevision, Oxehealth, Oxford, UK). The data collected from contact sensors were used by the polysomnography scorer to determine the 'true' input labels 20 for the epochs of the training data 10. However, the data from the contact sensors were not used to train the machine-learning algorithms. Rather, the training data 10 were derived from the simultaneously-acquired video images.

The PSG records were reviewed by a board-certified sleep specialist. Sleep stages were scored according to the AASM manual for sleep classification [1]. On the basis of its scoring rules, sleep recordings are divided into 6 discrete stages (wake, N1, N2, N3, REM, and movement). A total of 8 nights of overnight recordings were collected, with 2 of the 6 subjects completing two nights. One session was excluded from analysis on the basis that accelerometer data and a labelled hypnogram were not available (SV-008 'test' subject). Epochs were excluded where the sleep scorer assigned a poor confidence to the sleep staging labels (labelled 'A' in the hypnogram).

Logistic regression, k-nearest neighbours, a random forest classifier, and a neural multilayer perceptron (MLP) were used as classification algorithms. Pre-built implementations from scikit-learn for Python 3.7 (Python Software Foundation) were used for each model. Model parameters choices were based on prior works [2, 3, 4, 5]. Parameters adopted are shown in Table I. Models were trained and tested using leave-one-out cross-validation. The classification performance of each algorithm across all feature sets considered was summarized using receiver operating characteristic (ROC) curves. Performance was evaluated using metrics of accuracy and Area under the curve (AUC) on an epoch-per-epoch basis in comparison with ground-truth.

TABLE 1: MODEL PARAMETERS

| Algorithm | Parameter | Value |
|---|---|---|
| Logistic regression | penalty | L2 |
| | solver | liblinear |
| k-nearest neighbours | # weights | distance |
| Random forest | # estimators | 100 |
| | maximum # features | 1.0 |
| | maximum depth | 10 |
| | min samples split | 10 |

(continued)

| Algorithm | Parameter | Value |
|---|---|---|
| | min samples leaf | 32 |
| MLP | solver | adam |
| | activation function | ReLu |
| | maximum # iterations | 2000 |
| | hidden layer sizes | [(15, 15, 15)] |
| | alpha | 0.01 |

An experiment was conducted in which the data from contact sensors traditionally used to classify sleep states was replaced by input data derived from video images of subject from cameras.

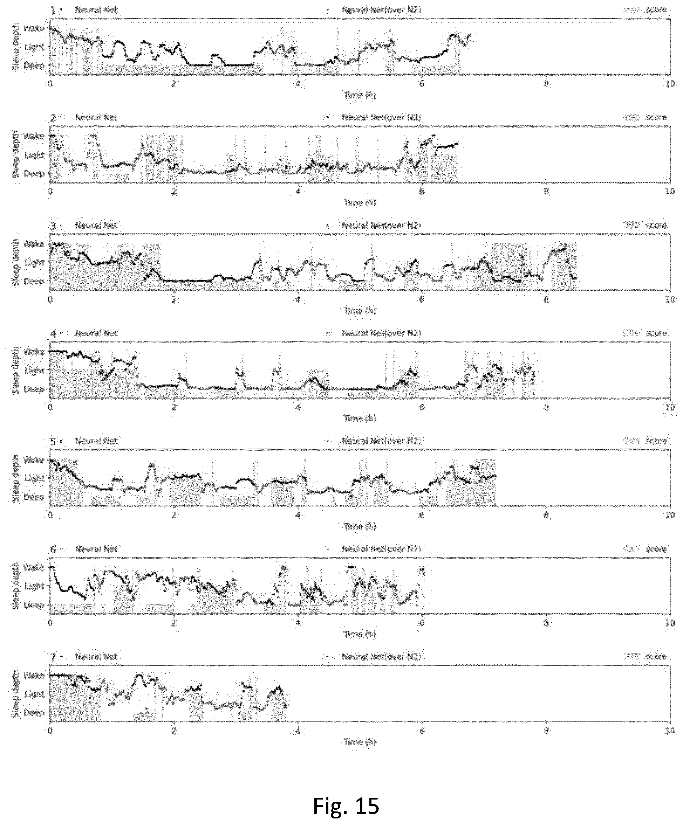

Fig. 15 shows sleep depth pseudo-hypnograms obtained for this experiment, in which all inputs to the MLP regressor are obtained from derived from video images of the subject. The training data and input data therefore comprised the measure of movement in a head region of the subject, the measure of movement in a torso region of the subject, the respiratory rate of the subject, and the variance of the respiratory rate of the subject. Pre-processing of the measures of movement was used to remove baseline wander, spurious peaks, and calibrate changes based on the initial wake period of each session. The respiratory rate for each epoch was calculated as the mean RR over a 4-minute time window centred on the epoch. The wake/light sleep/deep sleep labels derived from the expert assigned AASM labels are shown shaded in grey. Estimates of sleep depth are shown in black for the wake/light sleep/deep sleep epochs. N2-labelled epochs were excluded from the training data but included in the pseudo-hypnograms and shown in red.

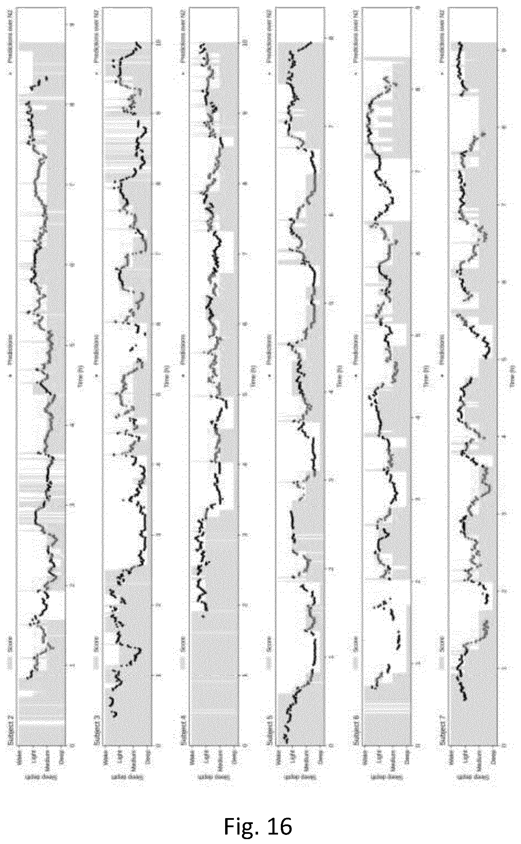

Fig. 16 shows similar sleep depth pseudo-hypnograms derived from a second experiment using a random forest machine-learning algorithm, but for the situation where an additional medium sleep training label is used, and intermediate NREM (N2) epochs are associated with the medium sleep training label. Light NREM sleep (N1) and REM sleep are still treated together as light sleep.

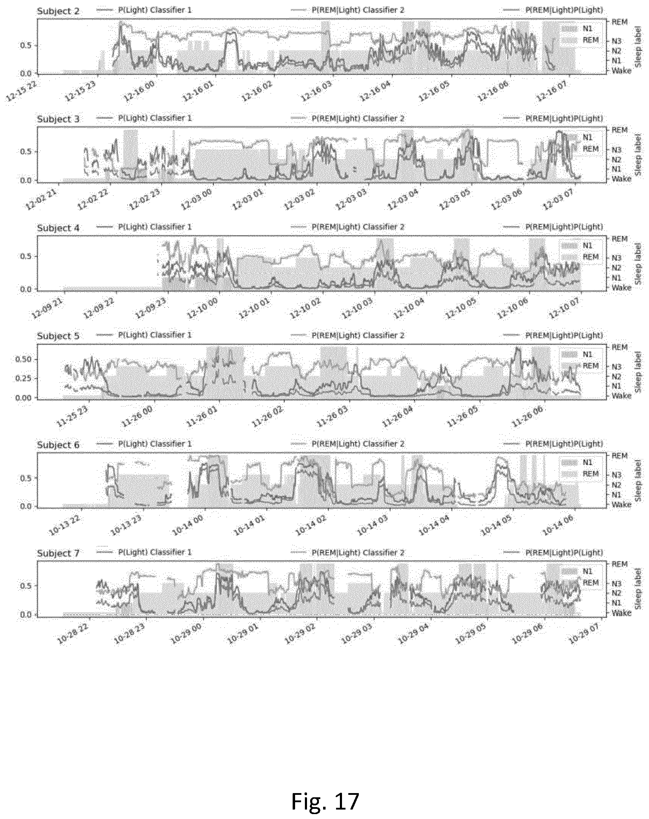

Fig. 17 shows results from a two-stage classification algorithm. The first stage classifies the sleep state as wake, light sleep (N1 and REM), medium sleep (N2), or deep sleep (N3). The second stage further classifies the sleep state as REM or NREM. p(Light) from the first stage classifier is shown in green, p(REMILight) from the second stage classifier is shown in blue, and their product p(Light)p(REMILight) is shown in orange. For subjects 4, 5, 6, and 7 there are clear spikes in the green signal (p(Light)p(REMILight)) around REM.

A further alternative implementation of the method of training a supervised machine-learning algorithm to determine a sleep state of a subject is to classify the sleep state directly into one of the desired state of output states, rather than using a multi-stage approach such as discussed above.

Similarly to the methods above, the method uses training data 10 and training labels 30 in respect of a plurality of subjects derived from video images of the subjects. The training data 10 comprises: a measure of subject movement; and a cardiorespiratory parameter of the subject. The method comprises training the supervised machine learning algorithm using the training data 10 and the training labels 30.

The training data 10 in respect of each subject is temporally divided into a plurality of epochs, each epoch being associated with a respective input label 20 indicating that a sleep state of the epoch is one of a set of sleep states comprising wake, rapid-eye movement (REM) sleep, light non-rapid eye movement (NREM) sleep, or deep NREM sleep.

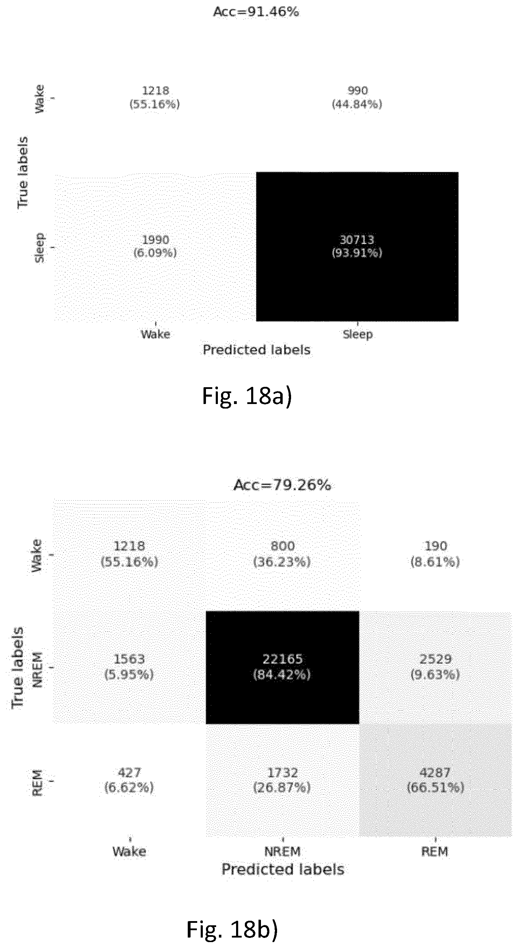

A first example is wake/sleep classification. In this case, the training labels 30 are selected from a set of training labels 30 indicating that the sleep state of the epoch is wake or sleep. In step S 10, epochs associated with an input label 20 indicating that the sleep state of the epoch is wake are associated with a training label 30 indicating that the sleep state of the epoch is wake. Epochs associated with an input label 20 indicating that the sleep state of the epoch is REM sleep, light NREM sleep, or deep NREM sleep are associated with a training label 30 indicating that the sleep state of the epoch is sleep. Fig. 18a) shows confusion matrices against the training label determined by a human scorer for this embodiment.

A second example is wake/REM/NREM classification. In this case, the training labels 30 are selected from a set of training labels 30 indicating that the sleep state of the epoch is wake, REM sleep, or NREM sleep. In step S 10, epochs associated with an input label 20 indicating that the sleep state of the epoch is wake are associated with a training label 30 indicating that the sleep state of the epoch is wake. Epochs associated with an input label 20 indicating that the sleep state of the epoch is REM sleep are associated with a training label 30 indicating that the sleep state of the epoch is REM sleep. Epochs associated with an input label 20 indicating that the sleep state of the epoch is light NREM sleep, or deep NREM sleep are associated with a training label (30) indicating that the sleep state of the epoch is NREM sleep. Fig. 18b) shows confusion matrices against the training label determined by a human scorer for this embodiment.

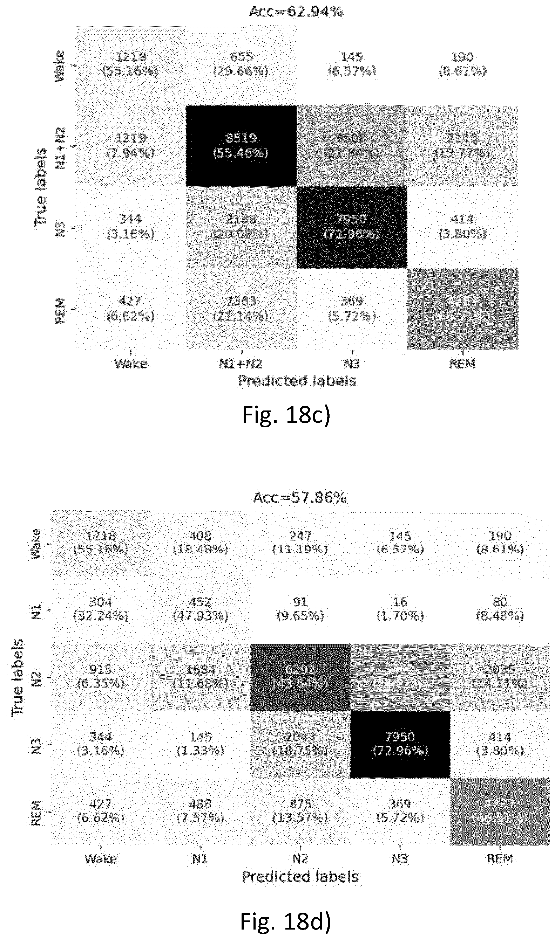

A third example is wake/REM/light NREM/deep NREM classification. In this case, the training labels 30 are selected from a set of training labels 30 indicating that the sleep state of the epoch is wake, REM sleep, light NREM sleep, or deep NREM sleep. In step S 10, epochs associated with an input label 20 indicating that the sleep state of the epoch is wake are associated with a training label 30 indicating that the sleep state of the epoch is wake. Epochs associated with an input label 20 indicating that the sleep state of the epoch is REM sleep are associated with a training label 30 indicating that the sleep state of the epoch is REM sleep. Epochs associated with an input label 20 indicating that the sleep state of the epoch is light NREM sleep are associated with a training label 30 indicating that the sleep state of the epoch is light NREM sleep. Epochs associated with an input label 20 indicating that the sleep state of the epoch is deep NREM sleep are associated with a training label 30 indicating that the sleep state of the epoch is deep NREM sleep. Fig. 18c) shows confusion matrices against the training label 30 determined by a human scorer for this embodiment.

In any of these first three examples, the set of sleep states indicated by the input labels 20 may further comprise intermediate NREM sleep. In this case, a proportion of the epochs associated with an input label 20 indicating that the sleep state of the epoch is intermediate NREM sleep are associated with the same training label 30 as epochs associated with an input label 20 indicating that the sleep state of the epoch is light NREM sleep. The proportion can be determined in any suitable manner such as discussed above.

Optionally, all of the epochs associated with an input label 20 indicating that the sleep state of the epoch is intermediate NREM sleep are associated with the same training label 30 as epochs associated with an input label 20 indicating that the sleep state of the epoch is light NREM sleep. This is the case for the results shown in Fig. 18a) to Fig. 18c), where light NREM sleep (N1) is combined with intermediate NREM sleep (N2) in each case.

Where the set of sleep states further comprises intermediate NREM sleep, the method may alternatively directly classify the sleep state into one of the full set of five sleep states. In this case, the training labels 30 are selected from a set of training labels 30 indicating that the sleep state of the epoch is wake, REM sleep, light NREM sleep, intermediate NREM sleep, or deep NREM sleep. In step S 10, epochs associated with an input label 20 indicating that the sleep state of the epoch is wake are associated with a training label 30 indicating that the sleep state of the epoch is wake. Epochs associated with an input label 20 indicating that the sleep state of the epoch is REM sleep are associated with a training label 30 indicating that the sleep state of the epoch is REM sleep. Epochs associated with an input label 20 indicating that the sleep state of the epoch is light NREM sleep are associated with a training label 30 indicating that the sleep state of the epoch is light NREM sleep. Epochs associated with an input label 20 indicating that the sleep state of the epoch is intermediate NREM sleep are associated with a training label 30 indicating that the sleep state of the epoch is intermediate NREM sleep. Epochs associated with an input label 20 indicating that the sleep state of the epoch is deep NREM sleep are associated with a training label 30 indicating that the sleep state of the epoch is deep NREM sleep. Fig. 18d) shows confusion matrices against the training label 30 determined by a human scorer for this embodiment.

Any suitable machine learning algorithm may be used to determine the sleep state, such as the examples of a regression or classification algorithm discussed above. The method may determine the sleep state using a continuous measure of sleep depth, or may classify the sleep state, again in any suitable manner such as described above.

Corresponding to this method of training a supervised machine-learning algorithm, there is also provided a method of determining a sleep state 60 of a test subject. The method comprises applying a machine-learning algorithm to input data 40 from the test subject. The input data 40 are derived from video images of the test subject, and the input data 40 comprises: a measure of subject movement; and a cardiorespiratory parameter of the subject. The video images can be analysed in the same way as described above for the training data 10 to obtain the input data 40 for the test subject.

The machine-learning algorithm outputs a determination of the sleep state of the test subject on the basis of training data 10 and training labels 30. The training data 10 is in respect of a plurality of training subjects and is derived from video images of the training subjects, as described above. The training data comprising for each of the training subjects: a measure of subject movement; and a cardiorespiratory parameter. The training data 10 and training labels 30 are otherwise as described above in relation to the training methods. There are two main option for the determination of the sleep state 60 of the test subject on the basis of the training data 10 and training labels 30. Which of these is used will depend on the type of machine-learning algorithm that is being used.

The first option is shown in Fig. 6, where the machine-learning algorithm is trained using a method of training a supervised machine learning algorithm, as discussed above. In this case, the machine-learning algorithm is pre-trained, and the input data 40 and trained machine-learning (ML) algorithm 50 are used together to determine S30 the sleep state 60. The input to step S30 corresponding to the trained ML algorithm 50 may comprise a plurality of parameters that were determined by the method of training the machine-learning algorithm. The input data 40 are fed into the trained machine-learning algorithm 50, which outputs the determination of the sleep state 60. Neural networks such as the multi-layer perceptron (whether as a regressor or classifier) are an example of this type of machine-learning algorithm.

The second option is shown in Fig. 7, where the machine-learning algorithm is of a type which is not pre-trained, but compares the input data 40 to the training data 10 at the time of being run. In this case, the relabelling S10 of the epochs in the training data 10 is carried out during the method, and the training data 10 are required to determine the sleep state for subsequent new input data 40 from test subjects.

Regardless of which option is used, the determination of the sleep state of the test subject may be a value of a continuous measure of sleep depth, or a classification of the sleep state, depending on the type of machine-learning algorithm used.

REFERENCES

[1] Richard B Berry, Rita Brooks, Charlene E Gamaldo, Susan M Harding, C Marcus, Bradley V Vaughn, et al. "The AASM manual for the scoring of sleep and associated events". In: Rules, Terminology and Technical Specifications, Darien, Illinois, American Academy of Sleep Medicine 176 (2019), p. 2019.
[2] Olivia Walch, Yitong Huang, Daniel Forger, and Cathy Goldstein. "Sleep stage prediction with raw acceleration and photoplethysmography heart rate data derived from a consumer wearable device". In: Sleep 42.12 (2019), zsz 180.
[3] James Pardey, Stephen Roberts, Lionel Tarassenko, and John Stradling. "A new approach to the analysis of the human sleep/wakefulness continuum". In: Journal of sleep research 5.4 (1996), pp. 201-210.
[4] Daniel M Roberts, Margeaux M Schade, Gina M Mathew, Daniel Gartenberg, and Orfeu M Buxton. "Detecting sleep using heart rate and motion data from multisensor consumer-grade wearables, relative to wrist actigraphy and polysomnography". In: Sleep 43.7 (2020), zsaa045.
[5] Mustafa Radha, Pedro Fonseca, Arnaud Moreau, Marco Ross, Andreas Cerny, Peter Anderer, Xi Long, and Ronald M Aarts. "Sleep stage classification from heart-rate variability using long short-term memory neural networks". In: Scientific reports 9.1 (2019), pp. 1-11.

Aspects of the invention may also be described by the following numbered clauses. These are not the claims of the application, which following under the heading "CLAIMS".

1. A method of training a supervised machine-learning algorithm to determine a sleep state of a subject, wherein the method uses training data in respect of a plurality of subjects derived from video images of the subjects, the training data in respect of each subject being temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep, and the method comprises training the supervised machine learning algorithm using the training data and a training label associated with each epoch selected from a set of training labels comprising training labels indicating that the sleep state of the epoch is wake, light sleep, or deep sleep, wherein epochs associated with an input label indicating that the sleep state of the epoch is wake are associated with a training label indicating that the sleep state of the epoch is wake, epochs associated with an input label indicating that the sleep state of the epoch is REM sleep or light NREM sleep are associated with a training label indicating that the sleep state of the epoch is light sleep, and epochs associated with an input label indicating that the sleep state of the epoch is deep NREM sleep are associated with a training label indicating that the sleep state of the epoch is deep sleep.

2. A method according to clause 1, wherein the set of sleep states further comprises intermediate NREM sleep, and a proportion of the epochs associated with an input label indicating that the sleep state of the epoch is intermediate NREM sleep are associated with a training label indicating that the sleep state of the epoch is light sleep.

3. A method according to clause 2, wherein the proportion of the epochs associated with an input label indicating that the sleep state of the epoch is intermediate NREM sleep comprises epochs associated with an input label indicating that the sleep state of the epoch is intermediate NREM sleep that immediately precede or follow an epoch associated with an input label indicating that the sleep state of the epoch is light NREM sleep.

4. A method according to any one of clauses 1 to 3, wherein the set of training labels is a set of three training labels comprising the training labels indicating that the sleep state of the epoch is wake, light sleep, or deep sleep.

5. A method according to clause 1, wherein: the set of sleep states further comprises intermediate NREM sleep; the set of training labels further comprises a training label indicating that the sleep state of the epoch is medium sleep; and epochs associated with an input label indicating that the sleep state of the epoch is intermediate NREM sleep are associated with a training label indicating that the sleep state of the epoch is medium sleep.

6. A method according to any one of clauses 1 to 5, wherein the machine learning algorithm is a regression algorithm, and the supervised machine-learning algorithm determines the sleep state using a continuous measure of sleep depth.

7. A method according to clause 6, wherein the regression algorithm is a neural network.

8. A method according to any one of clauses 1 to 5, wherein the machine learning algorithm is a classification algorithm that classifies the sleep state.

9. A method according to any one of clauses 1 to 5, wherein: the machine learning algorithm is a classification algorithm; the classification algorithm generates scores for each of the training labels representing a confidence of the sleep state being the sleep state indicated by the training label; and the machine-learning algorithm determines the sleep state using a continuous measure of sleep depth, the continuous measure being determined from a weighted combination of the scores for each of the training labels.

10. A method according to clause 8 or 9, wherein the classification algorithm is one of a logistic regression, k-nearest neighbours, a random forest classifier, and a neural network.

11. A method according to any one of clauses 1 to 10, wherein the training data comprise at least one feature derived from the video images of the subjects.

12. A method according to clause 11, wherein the at least one feature comprises a measure of subject movement, for example a measure of subject movement in a head region of the subject and/or a measure of movement in a torso region of the subject.

13. A method according to clause 11 or 12, wherein the at least one feature comprises a cardiorespiratory parameter of the subject, for example a respiratory rate of the subject, a variance of the respiratory rate of the subject, a heart rate of the subject, and/or a variance of the heart rate of the subject.

14. A method of training a supervised machine-learning algorithm to determine a sleep state of a subject, wherein the machine-learning algorithm comprises a first-stage classification algorithm and a second-stage classification algorithm, the method comprising: training the first-stage classification algorithm according to the method of clause 8 or any clause dependent thereon; training the second stage classification algorithm using the training data to further classify the sleep state as NREM sleep or REM sleep when the first stage classification algorithm classifies the sleep state as light sleep.

15. A method according to clause 14, wherein training the second stage classification algorithm comprises training the second stage classification algorithm using the training data and a second-stage training label associated with each epoch selected from a set of second-stage training labels comprising second-stage training labels indicating that the sleep state of the epoch is NREM sleep or REM sleep, wherein epochs associated with an input label indicating that the sleep state of the epoch is light NREM sleep are associated with a second-stage training label indicating that the sleep state of the epoch is NREM sleep.

16. A method according to clause 15, wherein epochs associated with an input label indicating that the sleep state of the epoch is a sleep state other than REM sleep are associated with a second-stage training label indicating that the sleep state of the epoch is NREM sleep.

17. A method according to any one of clauses 14 to 16, wherein the training data comprise at least one feature derived from the video images of the subjects.

18. A method according to clause 17, wherein the at least one feature comprises a measure of subject movement, for example a measure of subject movement in a head region of the subject and/or a measure of movement in a torso region of the subject.

19. A method according to clause 17 or 18, wherein the at least one feature comprises a cardiorespiratory parameter of the subject, for example a respiratory rate of the subject, a variance of the respiratory rate of the subject, a heart rate of the subject, and/or a variance of the heart rate of the subject.

20. A method according to any one of clauses 17 to 19, wherein at least one of the features is derived as a rolling average.

21. A method of determining a sleep state of a test subject comprising applying a machine-learning algorithm to input data from the test subject, wherein: the input data are derived from video images of the test subject; and the machine-learning algorithm outputs a determination of the sleep state of the test subject on the basis of training data and training labels, wherein the training data is in respect of a plurality of training subjects and is derived from video images of the training subjects, the training data in respect of each training subject being temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep, the training labels are associated with each epoch and selected from a set of training labels comprising training labels indicating that the sleep state of the epoch is wake, light sleep, or deep sleep, wherein epochs associated with an input label indicating that the sleep state of the epoch is wake are associated with a training label indicating that the sleep state of the epoch is wake, epochs associated with an input label indicating that the sleep state of the epoch is REM sleep or light NREM sleep are associated with a training label indicating that the sleep state of the epoch is light sleep, and epochs associated with an input label indicating that the sleep state of the epoch is deep NREM sleep are associated with a training label indicating that the sleep state of the epoch is deep sleep.

22. A method according to clause 21, wherein the machine-learning algorithm is trained using a method according to any one of clauses 1 to 13.

23. A method according to clause 21 or 22, wherein the input data comprise at least one feature derived from the video images of the test subject.

24. A method according to clause 23, wherein the at least one feature comprises a measure of subject movement, for example a measure of subject movement in a head region of the subject, and/or a measure of movement in a torso region of the subject.

25. A method according to clause 23 or 24, wherein the at least one feature comprises a cardiorespiratory parameter of the subject, for example a respiratory rate of the subject, and a variance of the respiratory rate of the subject, a heart rate of the subject, and/or a variance of the heart rate of the subject.

26. A method according to any one of clauses 21 to 25, wherein the set of training labels is a set of three training labels comprising the training labels indicating that the sleep state of the epoch is wake, light sleep, or deep sleep.

27. A method according to any one of clauses 21 to 25, wherein: the set of sleep states further comprises intermediate NREM sleep; the set of training labels further comprises a training label indicating that the sleep state of the epoch is medium sleep; epochs associated with an input label indicating that the sleep state of the epoch is intermediate NREM sleep are associated with a training label indicating that the sleep state of the epoch is medium sleep; and

28. A method according to any one of clauses 21 to 27, wherein the determination of the sleep state of the test subject is a value of a continuous measure of sleep depth.

29. A method according to clause 28, wherein the machine learning algorithm is trained using a method according to any one of clauses 6, 7, or 9.

30. A method according to any one of clauses 21 to 27, wherein the determination of the sleep state of the test subject is a classification of the sleep state.

31. A method according to clause 30, wherein the machine learning algorithm is trained using a method according to clause 8 or any clause dependent thereon.

32. A method of determining a sleep state of a test subject comprising: determining a sleep state of the test subject using a method according to clause 30 or 31; and if the sleep state is determined to be light sleep, further classifying the light sleep as REM or NREM sleep.

33. A method according to clause 32, wherein the input data comprise one or both of a measure of subject movement and a cardiorespiratory parameter of the subject, and the further classification is carried out using the input data.

34. A method according to clause 32 or 33, wherein the further classification is carried out using a second stage machine-learning classification algorithm that outputs a classification of the sleep state as NREM sleep or REM sleep on the basis of the training data and input labels.

35. A method according to clause 32, wherein the second stage classification algorithm uses the training data and a second-stage training label associated with each epoch selected from a set of second-stage training labels comprising second-stage training labels indicating that the sleep state of the epoch is NREM sleep or REM sleep, wherein epochs associated with an input label indicating that the sleep state of the epoch is REM sleep are associated with a second-stage training label indicating that the sleep state of the epoch is REM sleep; and epochs associated with an input label indicating that the sleep state of the epoch is light NREM sleep are associated with a second-stage training label indicating that the sleep state of the epoch is NREM sleep.

36. A method according to clause 35, wherein epochs associated with an input label indicating that the sleep state of the epoch is a sleep state other than REM sleep are associated with a second-stage training label indicating that the sleep state of the epoch is NREM sleep.

37. A method of determining a sleep state of a test subject comprising applying a machine-learning algorithm trained using a method according to any one of clauses 14 to 19 to input data from the test subject, the input data derived from video images of the test subject.

38. A method according to any one of clauses 32 to 37, wherein the input data comprise at least one feature derived from the video images of the test subject.

39. A method according to clause 38, wherein the at least one feature comprises a measure of subject movement, for example a measure of subject movement in a head region of the subject and/or a measure of movement in a torso region of the subject.

40. A method according to clause 38 or 39, wherein the at least one feature comprises a cardiorespiratory parameter of the subject, for example a respiratory rate of the subject, a variance of the respiratory rate of the subject, a heart rate of the subject, and/or a variance of the heart rate of the subject.

41. A method according to any one of clauses 38 to 40, wherein at least one of the features is derived as a rolling average.

42. A method of training a supervised machine-learning algorithm to determine a sleep state of a subject, wherein the method uses training data in respect of a plurality of subjects derived from video images of the subjects, the training data in respect of each subject being temporally divided into a plurality of epochs, each epoch being associated with a respective input label indicating that a sleep state of the epoch is one of a set of sleep states comprising rapid-eye movement (REM) sleep and light non-rapid eye movement (NREM) sleep, and the method comprises training the supervised machine learning algorithm using the training data and a training label associated with each epoch selected from a set of training labels comprising training labels indicating that the sleep state of the epoch is NREM sleep or REM sleep, wherein epochs associated with an input label indicating that the sleep state of the epoch is REM sleep are associated with a second-stage training label indicating that the sleep state of the epoch is REM sleep; and epochs associated with an input label indicating that the sleep state of the epoch is light NREM sleep are associated with a training label indicating that the sleep state of the epoch is NREM sleep.

43. A method according to clause 42, wherein epochs associated with an input label indicating that the sleep state of the epoch is a sleep state other than REM sleep are associated with a training label indicating that the sleep state of the epoch is NREM sleep.

44. A method according to clause 42 or 43, wherein the training data comprise at least one feature derived from the video images of the subjects.

45. A method according to clause 44, wherein the at least one feature comprises a measure of subject movement, for example a measure of subject movement in a head region of the subject and/or a measure of movement in a torso region of the subject.

46. A method according to clause 44 or 45, wherein the at least one feature comprises a cardiorespiratory parameter of the subject, for example a respiratory rate of the subject, a variance of the respiratory rate of the subject a heart rate of the subject, and/or a variance of the heart rate of the subject.

47. A method according to any one of clauses 44 to 46, wherein at least one of the features is derived as a rolling average.

48. A method of determining a sleep state of a test subject comprising applying a machine-learning algorithm to input data from the test subject, wherein the input data are derived from video images of the test subject; and the machine-learning algorithm outputs a determination of the sleep state of the test subject as light NREM sleep or REM sleep on the basis of training data and training labels, wherein the training data is in respect of a plurality of training subjects and is derived from video images of the training subjects, the training data in respect of each training subject being temporally divided into a plurality of epochs, each epoch being associated with a respective input label indicating that a sleep state of the epoch is one of a set of sleep states comprising rapid-eye movement (REM) sleep and light non-rapid eye movement (NREM) sleep; the training labels are associated with each epoch and selected from a set of training labels comprising training labels indicating that the sleep state of the epoch is NREM sleep or REM sleep, wherein epochs associated with an input label indicating that the sleep state of the epoch is REM sleep are associated with a second-stage training label indicating that the sleep state of the epoch is REM sleep; and epochs associated with an input label indicating that the sleep state of the epoch is light NREM sleep are associated with a training label indicating that the sleep state of the epoch is NREM sleep.

49. A method according to clause 48, wherein the machine-learning algorithm is trained using a method according to any one of clauses 42 to 47.

**Claims**

1. A method of training a supervised machine-learning algorithm to determine a sleep state of a subject, wherein:

   the method uses training data and training labels in respect of a plurality of subjects derived from video images of the subjects,
   the training data comprises: at least one measure of subject movement; and at least one cardiorespiratory parameter of the subject, and
   the method comprises training the supervised machine learning algorithm using the training data and the training labels.

2. A method according to claim 1, wherein the training data in respect of each subject is temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep.

3. A method according to claim 2, wherein:

   i) each epoch is associated with a respective one of the training labels, the training labels selected from a set of training labels indicating that the sleep state of the epoch is wake or sleep; epochs associated with an input label indicating that the sleep state of the epoch is wake are associated with a training label indicating that the sleep state of the epoch is wake; and epochs associated with an input label indicating that the sleep state of the epoch is REM sleep, light NREM sleep, or deep NREM sleep are associated with a training label indicating that the sleep state of the epoch is sleep;
or
ii) each epoch is associated with a respective one of the training labels, the training labels selected from a set of training labels indicating that the sleep state of the epoch is wake, REM sleep, or NREM sleep; epochs associated with an input label indicating that the sleep state of the epoch is wake are associated with a training label indicating that the sleep state of the epoch is wake; epochs associated with an input label indicating that the sleep state of the epoch is REM sleep are associated with a training label indicating that the sleep state of the epoch is REM sleep; and epochs associated with an input label indicating that the sleep state of the epoch is light NREM sleep, or deep NREM sleep are associated with a training label indicating that the sleep state of the epoch is NREM sleep;
or
iii) each epoch is associated with a respective one of the training labels, the training labels selected from a set of training labels indicating that the sleep state of the epoch is wake, REM sleep, light NREM sleep, or deep NREM sleep; epochs associated with an input label indicating that the sleep state of the epoch is wake are associated with a training label indicating that the sleep state of the epoch is wake; epochs associated with an input label indicating that the sleep state of the epoch is REM sleep are associated with a training label indicating that the sleep state of the epoch is REM sleep; epochs associated with an input label indicating that the sleep state of the epoch is light NREM sleep are associated with a training label indicating that the sleep state of the epoch is light NREM sleep; and epochs associated with an input label indicating that the sleep state of the epoch is deep NREM sleep are associated with a training label indicating that the sleep state of the epoch is deep NREM sleep;
optionally wherein in any of i) to iii), the set of sleep states further comprises intermediate NREM sleep, and a proportion of the epochs associated with an input label indicating that the sleep state of the epoch is intermediate NREM sleep are associated with the same training label as epochs associated with an input label indicating that the sleep state of the epoch is light NREM sleep.

4. A method according to claim 2, wherein either:

i) the set of sleep states further comprises intermediate NREM sleep; each epoch is associated with a respective one of the training labels, the training labels selected from a set of training labels indicating that the sleep state of the epoch is wake, REM sleep, light NREM sleep, intermediate NREM sleep, or deep NREM sleep; epochs associated with an input label indicating that the sleep state of the epoch is wake are associated with a training label indicating that the sleep state of the epoch is wake; epochs associated with an input label indicating that the sleep state of the epoch is REM sleep are associated with a training label indicating that the sleep state of the epoch is REM sleep; epochs associated with an input label indicating that the sleep state of the epoch is light NREM sleep are associated with a training label indicating that the sleep state of the epoch is light NREM sleep; epochs associated with an input label indicating that the sleep state of the epoch is intermediate NREM sleep are associated with a training label indicating that the sleep state of the epoch is intermediate NREM sleep; and epochs associated with an input label indicating that the sleep state of the epoch is deep NREM sleep are associated with a training label indicating that the sleep state of the epoch is deep NREM sleep; or
ii) each epoch is associated with a respective one of the training labels, the training labels selected from a set of training labels indicating that the sleep state of the epoch is wake, light sleep, or deep sleep; epochs associated with an input label indicating that the sleep state of the epoch is wake are associated with a training label indicating that the sleep state of the epoch is wake; epochs associated with an input label indicating that the sleep state of the epoch is REM sleep or light NREM sleep are associated with a training label indicating that the sleep state of the epoch is light sleep; epochs associated with an input label indicating that the sleep state of the epoch is deep NREM sleep are associated with a training label indicating that the sleep state of the epoch is deep sleep, optionally wherein the set of sleep states further comprises intermediate NREM sleep, and a proportion of the epochs associated with an input label indicating that the sleep state of the epoch is intermediate NREM sleep are associated with a training label indicating that the sleep state of the epoch is light sleep.

5. A method according to any one of the preceding claims, wherein:

i) the machine learning algorithm is a regression algorithm, and the supervised machine-learning algorithm determines the sleep state using a continuous measure of sleep depth, optionally wherein the regression algorithm is a neural network; or
ii) the machine learning algorithm is a classification algorithm that classifies the sleep state, optionally wherein the classification algorithm is one of a logistic regression, k-nearest neighbours, a random forest classifier, and a neural network; or

iii) the machine learning algorithm is a classification algorithm, optionally one of a logistic regression, k-nearest neighbours, a random forest classifier, and a neural network; the classification algorithm generates scores for each of the training labels representing a confidence of the sleep state being the sleep state indicated by the training label; and the machine-learning algorithm determines the sleep state using a continuous measure of sleep depth, the continuous measure being determined from a weighted combination of the scores for each of the training labels.

6. A method according to any one of the preceding claims, wherein:

i) the at least one measure of subject movement comprises a measure of subject movement in a head region of the subject and/or a measure of movement in a torso region of the subject; and/or

ii) the at least one cardiorespiratory parameter of the subject comprises a respiratory rate of the subject, a variance of the respiratory rate of the subject, a heart rate of the subject, and/or a variance of the heart rate of the subject; and/or

iii) the measure of subject movement and/or the cardiorespiratory parameter of the subject is derived as a rolling average.

7. A method according to any one of the preceding claims, further comprising deriving the at least one measure of subject movement and the at least one cardiorespiratory parameter of the subject from the video images of the subjects,

optionally wherein the step of deriving the at least one cardiorespiratory parameter of the subject comprises deriving heart rate and respiratory rate from the video images of the subjects and deriving the at least one cardiorespiratory parameter from the heart rate and respiratory rate.

8. A method of determining a sleep state of a test subject comprising applying a machine-learning algorithm to input data from the test subject, wherein:

the input data are derived from video images of the test subject; and

the input data comprises: at least one measure of subject movement; and at least one cardiorespiratory parameter of the subject; and

the machine-learning algorithm outputs a determination of the sleep state of the test subject on the basis of training data and training labels,

wherein the training data is in respect of a plurality of training subjects and is derived from video images of the training subjects, the training data comprising for each of the training subjects: at least one measure of subject movement; and at least one cardiorespiratory parameter.

9. A method according to claim 8, wherein the training data in respect of each training subject is temporally divided into a plurality of epochs, each epoch being associated with a respective input label indicating that a sleep state of the epoch is one of a set of sleep states comprising wake, rapid-eye movement (REM) sleep, light non-rapid eye movement (NREM) sleep, or deep NREM sleep.

10. A method according to claim 9, wherein:

i) each epoch is associated with a respective one of the training labels, the training labels selected from a set of training labels indicating that the sleep state of the epoch is wake or sleep; epochs associated with an input label indicating that the sleep state of the epoch is wake are associated with a training label indicating that the sleep state of the epoch is wake; and epochs associated with an input label indicating that the sleep state of the epoch is REM sleep, light NREM sleep, or deep NREM sleep are associated with a training label indicating that the sleep state of the epoch is sleep;

or

ii) each epoch is associated with a respective one of the training labels, the training labels selected from a set of training labels indicating that the sleep state of the epoch is wake, REM sleep, or NREM sleep, wherein epochs associated with an input label indicating that the sleep state of the epoch is wake are associated with a training label indicating that the sleep state of the epoch is wake, epochs associated with an input label indicating that the sleep state of the epoch is REM sleep are associated with a training label indicating that the sleep state of the epoch is REM sleep, epochs associated with an input label indicating that the sleep state of the epoch is light NREM sleep, or deep NREM sleep are associated with a training label indicating that the sleep state of the epoch is NREM sleep;

or

iii) each epoch is associated with a respective one of the training labels, the training labels selected from a set of training labels indicating that the sleep state of the epoch is wake, REM sleep, light NREM sleep, or deep NREM sleep, wherein epochs associated with an input label indicating that the sleep state of the epoch is wake are associated with a training label indicating that the sleep state of the epoch is wake, epochs associated with an input label indicating that the sleep state of the epoch is REM sleep are associated with a training label indicating that the sleep state of the epoch is REM sleep, epochs associated with an input label indicating that the sleep state of the epoch is light NREM sleep are associated with a training label indicating that the sleep state of the epoch is light NREM sleep, and epochs associated with an input label indicating that the sleep state of the epoch is deep NREM sleep are associated with a training label indicating that the sleep state of the epoch is deep NREM sleep.

optionally wherein in any of i) to iii), the set of sleep states further comprises intermediate NREM sleep, and a proportion of the epochs associated with an input label indicating that the sleep state of the epoch is intermediate NREM sleep are associated with the same training label as epochs associated with an input label indicating that the sleep state of the epoch is light NREM sleep.

**11.** A method according to claim 9, wherein either:

i) the set of sleep states further comprises intermediate NREM sleep; each epoch is associated with a respective one of the training labels, the training labels selected from a set of training labels indicating that the sleep state of the epoch is wake, REM sleep, light NREM sleep, intermediate NREM sleep, or deep NREM sleep, wherein epochs associated with an input label indicating that the sleep state of the epoch is wake are associated with a training label indicating that the sleep state of the epoch is wake, epochs associated with an input label indicating that the sleep state of the epoch is REM sleep are associated with a training label indicating that the sleep state of the epoch is REM sleep, epochs associated with an input label indicating that the sleep state of the epoch is light NREM sleep are associated with a training label indicating that the sleep state of the epoch is light NREM sleep, epochs associated with an input label indicating that the sleep state of the epoch is intermediate NREM sleep are associated with a training label indicating that the sleep state of the epoch is intermediate NREM sleep, and epochs associated with an input label indicating that the sleep state of the epoch is deep NREM sleep are associated with a training label indicating that the sleep state of the epoch is deep NREM sleep; or

ii) each epoch is associated with a respective one of the training labels, the training labels selected from a set of training labels indicating that the sleep state of the epoch is wake, light sleep, or deep sleep, wherein epochs associated with an input label indicating that the sleep state of the epoch is wake are associated with a training label indicating that the sleep state of the epoch is wake, epochs associated with an input label indicating that the sleep state of the epoch is REM sleep or light NREM sleep are associated with a training label indicating that the sleep state of the epoch is light sleep, and epochs associated with an input label indicating that the sleep state of the epoch is deep NREM sleep are associated with a training label indicating that the sleep state of the epoch is deep sleep, optionally wherein the set of sleep states further comprises intermediate NREM sleep, and a proportion of the epochs associated with an input label indicating that the sleep state of the epoch is intermediate NREM sleep are associated with a training label indicating that the sleep state of the epoch is light sleep.

**12.** A method according to any one of claims 8 to 11, wherein:

i) the at least one measure of subject movement comprises a measure of subject movement in a head region of the subject, and/or a measure of movement in a torso region of the subject; and/or

ii) the at least one cardiorespiratory parameter of the subject comprises a respiratory rate of the subject, a variance of the respiratory rate of the subject, a heart rate of the subject, and/or a variance of the heart rate of the subject; and/or

iii) the measure of test subject movement and/or the cardiorespiratory parameter of the subject is derived as a rolling average.

**13.** A method according to any one of claims 8 to 12, wherein either:

i) the determination of the sleep state of the test subject is a value of a continuous measure of sleep depth, optionally wherein the machine learning algorithm is trained using a method according to claim 5 option i) or iii), or any preceding claim dependent thereon; or

ii) the determination of the sleep state of the test subject is a classification of the sleep state, optionally wherein the machine learning algorithm is trained using a method according to claim 5 option ii), or any preceding claim dependent thereon.

14. A method according to any one of claims 8 to 13, further comprising deriving the at least one measure of subject movement and the at least one cardiorespiratory parameter from the video images of the test subject, optionally wherein the step of deriving the at least one cardiorespiratory parameter of the subject comprises extracting heart rate and respiratory rate from the video images of the test subject, and deriving the at least one cardiorespiratory parameter from the heart rate and respiratory rate.

15. A computer program comprising, or a computer-readable storage medium having stored thereon, instructions which, when carried out by a computer, cause the computer to carry out the method of any preceding claim.

Fig. 1

Fig. 2

Fig. 3

Fig. 4

Fig. 5

Fig. 6

Fig. 7

Fig. 8

Fig. 9

Fig. 10

10 — Training data

Input labels — 20

Re-label — S10

S22 — Train 1st stage

Training labels — 30

Train 2nd stage — S24

Trained ML algorithm — 50

Fig. 11

Fig. 12

Fig. 13

Fig. 14

Fig. 15

Fig. 16

Fig. 17

Acc=91.46%

Fig. 18a)

Acc=79.26%

Fig. 18b)

Acc=62.94%

Fig. 18c)

Acc=57.86%

Fig. 18d)

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/152551 A1 (RESMED SENSOR TECH LTD [IE]) 5 August 2021 (2021-08-05)<br>* abstract *<br>* paragraph [0083] *<br>* paragraph [0096] *<br>* paragraph [0111] *<br>* paragraph [0123] *<br>* paragraph [0152] *<br>----- | 1-15 | INV.<br>A61B5/00<br>A61B5/11<br>A61B5/024 |
| A | US 2021/275090 A1 (PAPINI GABRIELE [NL] ET AL) 9 September 2021 (2021-09-09)<br>* abstract *<br>* paragraph [0057] *<br>* paragraph [0060] *<br>* paragraph [0088] *<br>----- | 1-15 | |
| A | WATSON NATHANIEL F ET AL: "Artificial intelligence and sleep: Advancing sleep medicine",<br>SLEEP MEDICINE REVIEWS, W.B. SAUNDERS, AMSTERDAM, NL,<br>vol. 59, 2 June 2021 (2021-06-02),<br>XP086784775,<br>ISSN: 1087-0792, DOI: 10.1016/J.SMRV.2021.101512<br>[retrieved on 2021-06-02]<br>* abstract *<br>* page 6, left-hand column, line 25 - line 30 *<br>----- | 1-15 | TECHNICAL FIELDS SEARCHED (IPC)<br><br>A61B |

-/--

The present search report has been drawn up for all claims

| Place of search | Date of completion of the search | Examiner |
|---|---|---|
| The Hague | 21 April 2023 | Dhondt, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 23 15 5317

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NOCHINO TERUAKI ET AL: "Sleep stage estimation method using a camera for home use", BIOMEDICAL ENGINEERING LETTERS, THE KOREAN SOCIETY OF MEDICAL AND BIOLOGICAL ENGINEERING, KOREA, vol. 9, no. 2, 24 April 2019 (2019-04-24), pages 257-265, XP036784118, ISSN: 2093-9868, DOI: 10.1007/S13534-019-00108-W [retrieved on 2019-04-24] * abstract; figures 1,3 * ----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 21 April 2023 | Dhondt, Erik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**page 2 of 2**

# EP 4 223 214 A1

## ANNEX TO THE EUROPEAN SEARCH REPORT
## ON EUROPEAN PATENT APPLICATION NO.

EP 23 15 5317

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2023

| Patent document cited in search report | | Publication date | Patent family member(s) | | Publication date |
|---|---|---|---|---|---|
| WO 2021152551 | A1 | 05-08-2021 | AU | 2021212395 A1 | 25-08-2022 |
| | | | CN | 115334959 A | 11-11-2022 |
| | | | EP | 4096503 A1 | 07-12-2022 |
| | | | JP | 2023513888 A | 04-04-2023 |
| | | | US | 2023099622 A1 | 30-03-2023 |
| | | | WO | 2021152551 A1 | 05-08-2021 |
| US 2021275090 | A1 | 09-09-2021 | CN | 115209801 A | 18-10-2022 |
| | | | EP | 3875026 A1 | 08-09-2021 |
| | | | EP | 4114264 A1 | 11-01-2023 |
| | | | US | 2021275090 A1 | 09-09-2021 |
| | | | WO | 2021175630 A1 | 10-09-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 19220087

### Non-patent literature cited in the description

- The AASM manual for the scoring of sleep and associated events. **RICHARD B BERRY ; RITA BROOKS ; CHARLENE E GAMALDO ; SUSAN M HARDING ; C MARCUS ; BRADLEY V VAUGHN et al.** Rules, Terminology and Technical Specifications. American Academy of Sleep Medicine, 2019, vol. 176, 2019
- **OLIVIA WALCH ; YITONG HUANG ; DANIEL FORGER ; CATHY GOLDSTEIN.** Sleep stage prediction with raw acceleration and photoplethysmography heart rate data derived from a consumer wearable device. *Sleep,* 2019, vol. 42 (12), 180
- **JAMES PARDEY ; STEPHEN ROBERTS ; LIONEL TARASSENKO ; JOHN STRADLING.** A new approach to the analysis of the human sleep/wakefulness continuum. *Journal of sleep research,* 1996, vol. 5 (4), 201-210
- **DANIEL M ROBERTS ; MARGEAUX M SCHADE ; GINA M MATHEW ; DANIEL GARTENBERG ; ORFEU M BUXTON.** Detecting sleep using heart rate and motion data from multisensor consumer-grade wearables, relative to wrist actigraphy and polysomnography. *Sleep,* 2020, vol. 43 (7), 045
- **MUSTAFA RADHA ; PEDRO FONSECA ; ARNAUD MOREAU ; MARCO ROSS ; ANDREAS CERNY ; PETER ANDERER ; XI LONG ; RONALD M AARTS.** Sleep stage classification from heart-rate variability using long short-term memory neural networks. *Scientific reports,* 2019, vol. 9 (1), 1-11